# EUROPEAN PATENT APPLICATION

(11) **EP 4 722 238 A1**
(43) Date of publication of application: **08.04.2026**
(21) Application number: 24831099.7
(22) Date of filing: 23.08.2024
(51) Int. Cl.: C07K 14/725, C07K 16/28, A61P 35/00, A61K 39/00, C07K 19/00, C12N 5/10

(54) **CHIMERIC ANTIGEN RECEPTOR BASED ON INTRACELLULAR REGION TRUNCATION**

(30) Priority: 30.06.2023 CN 202310803204
(71) Applicant: Center for Excellence in Molecular Cell Science, Chinese Academy of Sciences, Shanghai 200031 (CN)
(72) Inventor: XU, Chenqi, Shanghai 200031 (CN); XU, Xinyi, Shanghai 200031 (CN); XU, Xiaomin, Shanghai 200031 (CN); CHEN, Haotian, Shanghai 200031 (CN); WU, Wei, Shanghai 200031 (CN)
(74) Representative: Canzler & Bergmeier Patentanwälte Partnerschaft mbB
(86) International application number: PCT/CN2024/114101
(87) International publication number: WO 2025/002479

(57) **Abstract**

Provided is a chimeric antigen receptor based on an intracellular region truncation. The chimeric antigen receptor comprises an extracellular domain, a transmembrane domain and an intracellular domain which are linked in sequence, wherein the extracellular domain comprises an antigen recognition region and a hinge region, and the intracellular domain comprises a CD3ε intracellular region truncation, a costimulatory signaling region and a CD3ζ intracellular region which are linked in sequence. Compared with the prior art, the chimeric antigen receptor provided has a better efficacy and fewer side effects, which are reflected in the increased level of CAR molecules on the membrane surface of CAR-T cells, the formation of better immunological synapses, a better antigen sensitivity, fewer cytokines, a better growth ability, a better sustained proliferation ability, a better repetitive killing ability, attenuated transformation into NK cells and attenuated cell depletion, and the improved stemness and memory cell subset of CAR-T. Also, the chimeric antigen receptor can further improve the therapeutic effects on tumors. In addition, the chimeric antigen receptor down-regulates the cytokines thereof to reduce inflammatory cytokines produced by macrophage and monocyte activation, thereby preventing cytokine storms in the early stage.

## Description

### FIELD OF TECHNOLOGY

The present disclosure relates to the technical field of chimeric antigen receptors, and in particular to a chimeric antigen receptor based on a truncated intracellular region.

### BACKGROUND

The chimeric antigen receptor is abbreviated as CAR. T cells equipped with a CAR targeting a specific tumor antigen are referred to as CAR-T cells. CAR-T therapy is widely used in tumor treatment; however, it still faces multiple limitations. For example, in clinical treatment of leukemia and solid tumors, CAR-T therapy is often associated with cytokine storm, NK-like differentiation of CAR-T cells, rapid onset of T cell exhaustion, and suboptimal therapeutic efficacy, especially in the context of solid-tumor immunotherapy.

Prior art CN112500492A reported modifications to the sequentially connected intracellular domain and transmembrane domain of a CAR. It was found that when the intracellular region was arranged as: CD3ε intracellular region + costimulatory signaling intracellular region + CD3ζ intracellular region, the resulting CAR exhibited good antitumor efficacy with fewer adverse effects. However, this CAR showed reduced membrane surface expression, was prone to NK-like differentiation and T cell exhaustion, and required further improvement in growth capacity, sustained proliferation, cytotoxicity, and antigen sensitivity.

Accordingly, there is a need for a chimeric antigen receptor with improved therapeutic efficacy and reduced adverse effects, thereby providing new strategies for CAR-T therapy.

### SUMMARY

The objective of the present disclosure is to provide a chimeric antigen receptor and a use thereof.

To achieve the above objective and other related objectives, the first aspect of the present disclosure provides a chimeric antigen receptor, including:
an extracellular domain, a transmembrane domain, and an intracellular domain, which are sequentially connected;
wherein the extracellular domain includes an antigen-binding region and a hinge region; and
wherein the intracellular domain includes, in sequence, a truncated CD3ε intracellular region, a costimulatory signaling region, and a CD3ζ intracellular region.

The second aspect of the present disclosure provides a polynucleotide sequence, including:
(1) a polynucleotide sequence encoding the foregoing chimeric antigen receptor; and/or
(2) the complementary sequence of the polynucleotide sequence described in (1).

The third aspect of the present disclosure provides a nucleic acid construct, including the foregoing polynucleotide sequence.

The fourth aspect of the present disclosure provides a lentiviral vector system, including the foregoing nucleic acid construct and a lentiviral auxiliary component.

The fifth aspect of the present disclosure provides a genetically modified T cell, including the foregoing polynucleotide sequence or the foregoing nucleic acid construct, or being infected with the foregoing lentiviral vector system.

The sixth aspect of the present disclosure provides a use of the foregoing chimeric antigen receptor, or the foregoing polynucleotide sequence, or the foregoing nucleic acid construct, or the foregoing lentiviral vector system, in the preparation of a product for one or more of the following purposes: (1) preparing CAR-T cells; (2) increasing surface expression level of CAR on CAR-T cells; (3) enhancing antigen sensitivity of CAR-T cells; (4) suppressing IFN-y, IL-2, and TNF cytokine secretion of CAR-T cells; (5) promoting natural growth of CAR-T cells under resting conditions; (6) enhancing sustained proliferation of CAR-T cells after stimulation by target cells; (7) improving repetitive killing capacity of CAR-T cells; (8) reducing NK-like differentiation and exhaustion of CAR-T cells; (9) increasing stemness and memory cell subsets of CAR-T cells.

The seventh aspect of the present disclosure provides a use of the foregoing chimeric antigen receptor, or the foregoing polynucleotide sequence, or the foregoing nucleic acid construct, or the foregoing lentiviral vector system, or the foregoing genetically modified T cell, in the preparation of a product for tumor therapy.

As described above, the chimeric antigen receptor and the use thereof provided by the present disclosure offer the following beneficial effects:
1. Compared to the prior art, the chimeric antigen receptor of the present disclosure exhibits superior therapeutic efficacy and fewer side effects, as reflected in: increased surface expression level of CAR on CAR-T cells; enhanced formation of immunological synapses; improved antigen sensitivity; reduced cytokine secretion; improved cell growth capability; enhanced sustained proliferative capacity; superior repetitive killing capacity; reduced NK-like differentiation and T cell exhaustion; and increased stemness and memory cell subsets of CAR-T cells.
2. The chimeric antigen receptor of the present disclosure further enhances antitumor therapeutic efficacy. In addition, by downregulating its own cytokine production to reduce macrophage/monocyte activation and thereby lowering inflammatory cytokine release, the chimeric antigen receptor helps prevent cytokine storm at the early stage.

### BRIEF DESCRIPTION OF DRAWINGS

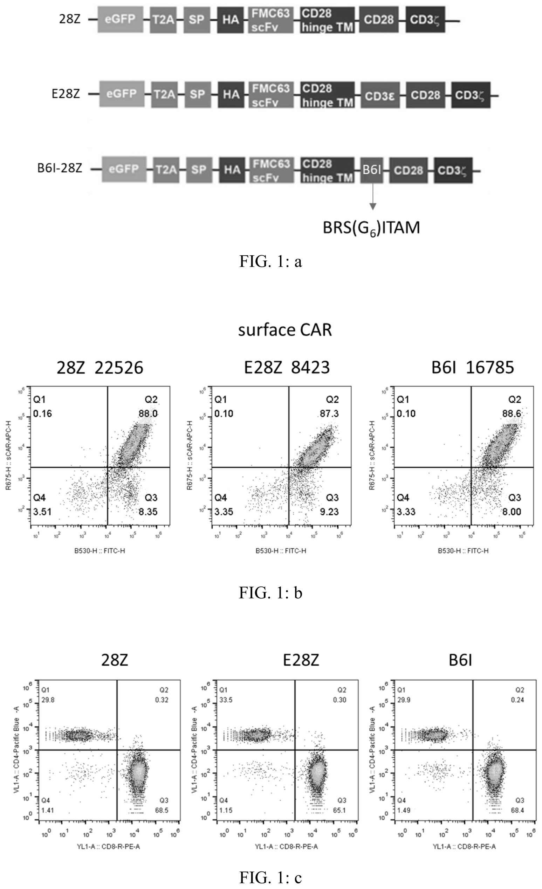

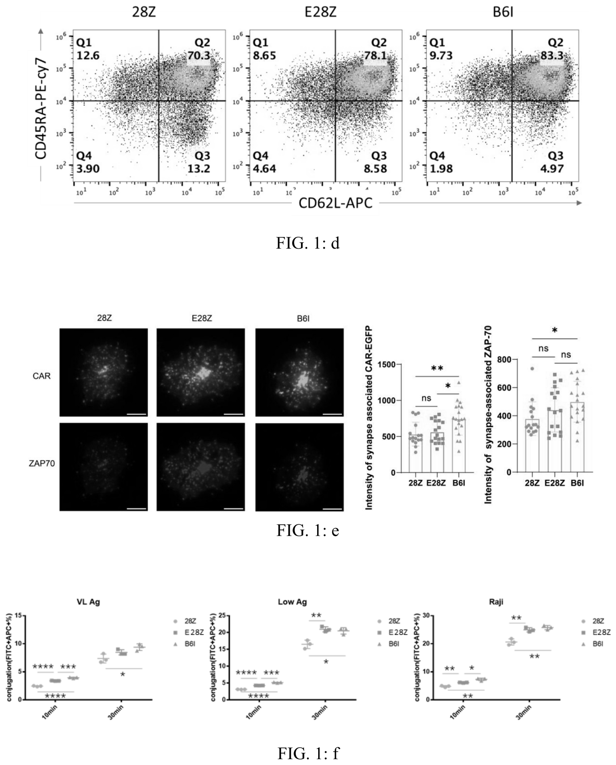

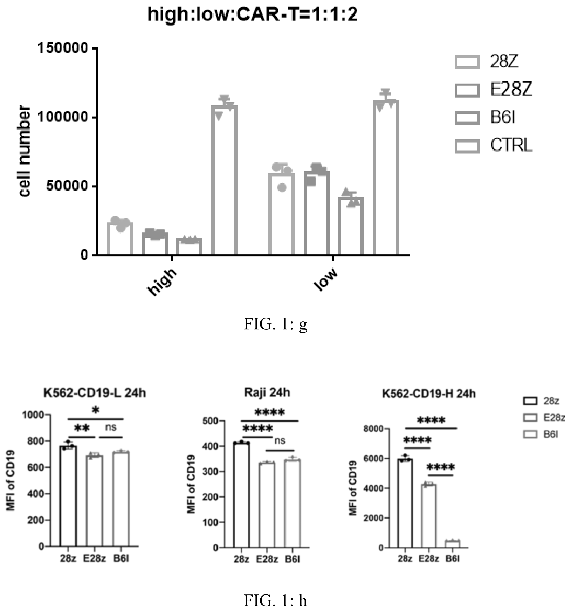

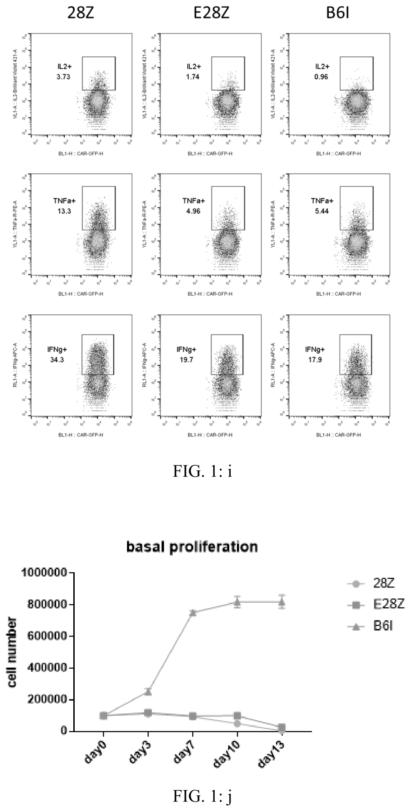

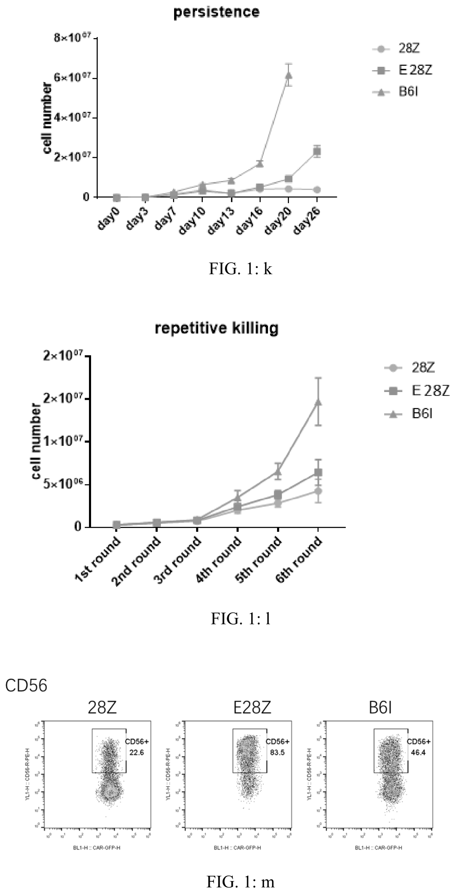

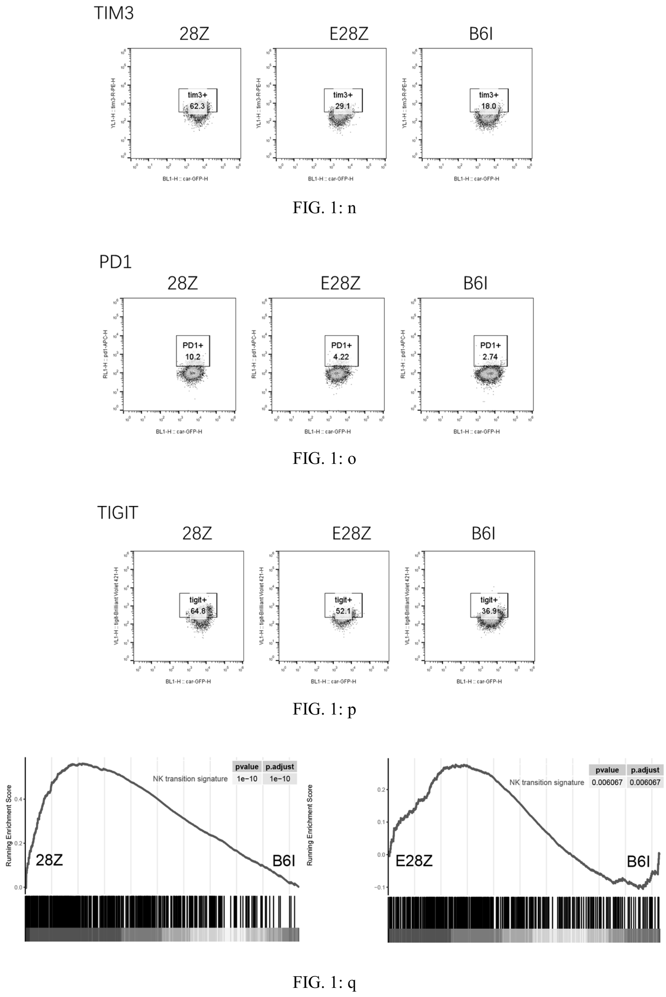

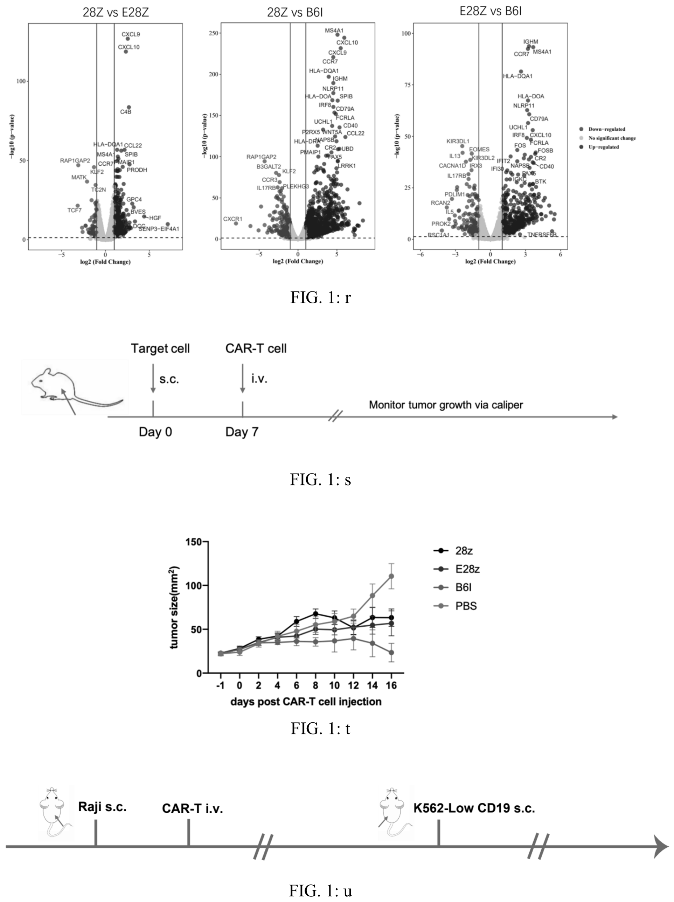

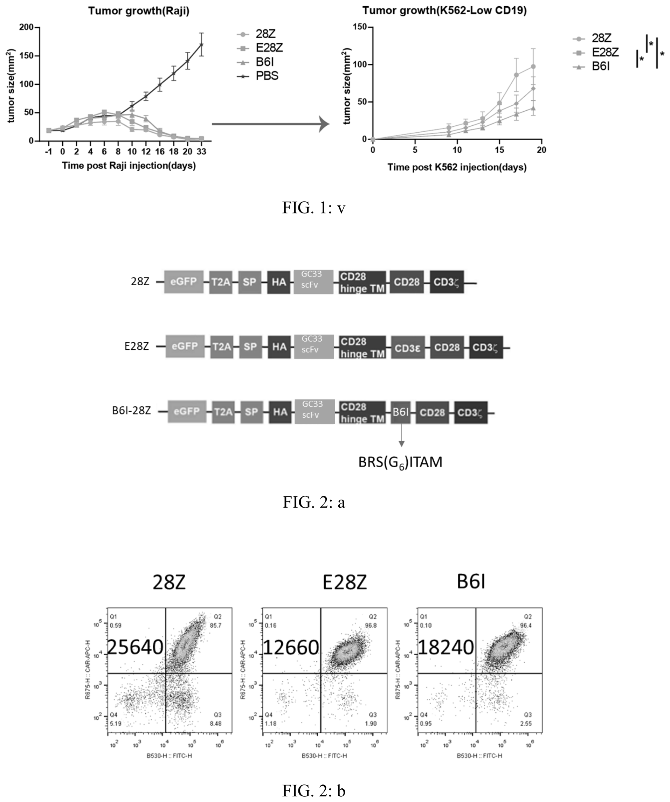

FIG. 1: a shows a schematic diagram of the structures of anti-CD19 28Z CAR, E28Z CAR, and B6I-28Z CAR.
FIG. 1: b shows flow cytometry plots of surface expression levels of anti-CD19 CAR on 28Z, E28Z, and B6I-28Z CAR-T cells.
FIG. 1: c shows flow cytometry plots of the CD4⁺ and CD8⁺ subset ratios in 28Z, E28Z, and B6I-28Z CAR-T cells.
FIG. 1: d shows flow cytometry plots of memory cell subsets in 28Z, E28Z, and B6I-28Z CAR-T cells under resting conditions.
FIG. 1: e shows immunological synapse images and ZAP70 recruitment capacity of anti-CD19 28Z, E28Z, and B6I-28Z CAR-T cells after CD19-antigen stimulation.
FIG. 1: f shows the binding capacity of anti-CD19 28Z, E28Z, and B6I-28Z CAR-T cells to target cells at different antigen densities.
FIG. 1: g shows the killing ability of anti-CD19 28Z, E28Z, and B6I-28Z CAR-T cells against target cells at different antigen densities.
FIG. 1: h shows membrane antigen expression levels of residual target cells after 24-hour co-incubation of anti-CD19 28Z, E28Z, and B6I-28Z CAR-T cells with target cells at different antigen densities.
FIG. 1: i shows cytokine secretion levels of anti-CD19 28Z, E28Z, and B6I-28Z CAR-T cells after CD19-antigen stimulation.
FIG. 1: j shows growth curves of anti-CD19 28Z, E28Z, and B6I-28Z CAR-T cells in the absence of antigen stimulation.
FIG. 1: k shows the sustained proliferative capacity of anti-CD19 28Z, E28Z, and B6I-28Z CAR-T cells after a single stimulation with Raji cells.
FIG. 1:1 shows the repetitive killing capability of anti-CD19 28Z, E28Z, and B6I-28Z CAR-T cells against Raji cells.
FIG. 1: m shows NK-like differentiation levels of anti-CD19 28Z, E28Z, and B6I-28Z CAR-T cells at the late stage of repetitive killing.
FIGs. 1: n-p show T cell exhaustion of anti-CD19 28Z, E28Z, and B6I-28Z CAR-T cells at the late stage of repetitive killing.
FIG. 1: q shows NK-like differentiation levels of anti-CD19 28Z, E28Z, and B6I-28Z CAR-T cells after five rounds of repetitive killing.
FIG. 1: r shows differential gene expression analysis results of anti-CD19 28Z, E28Z, and B6I-28Z CAR-T cells after five rounds of repetitive killing.
FIG. 1: s shows a flowchart of treating a Raji subcutaneous xenograft tumor model using CAR-T cells.
FIG. 1: t shows the growth of the Raji subcutaneous xenograft tumors after CAR-T cell treatment.
FIG. 1: u shows a flowchart of treating the Raji subcutaneous xenograft tumor model and tumor relapse model using CAR-T cells.
FIG. 1: v shows the growth of the Raji subcutaneous xenograft tumors and relapsed tumors after CAR-T cell treatment.
FIG. 2: a shows a schematic diagram of the structures of anti-GPC3 28Z CAR, E28Z CAR, and B6I-28Z CAR.

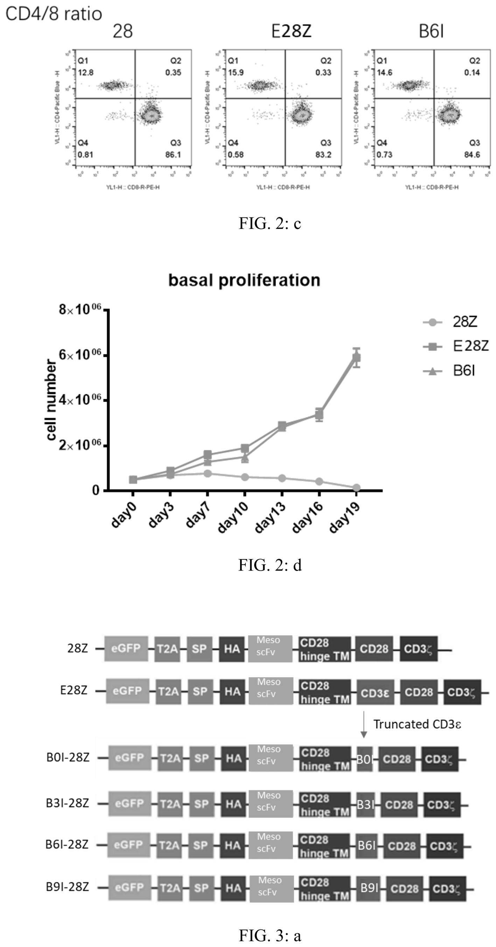

FIG. 2: b shows flow cytometry plots of surface expression levels of anti-GPC3 CAR on 28Z, E28Z, and B6I-28Z CAR-T cells.
FIG. 2: c shows flow cytometry plots of the CD4⁺ and CD8⁺ subset ratios in 28Z, E28Z, and B6I-28Z CAR-T cells.
FIG. 2: d shows growth curves of anti-GPC3 28Z, E28Z, and B6I-28Z CAR-T cells in the absence of antigen stimulation.

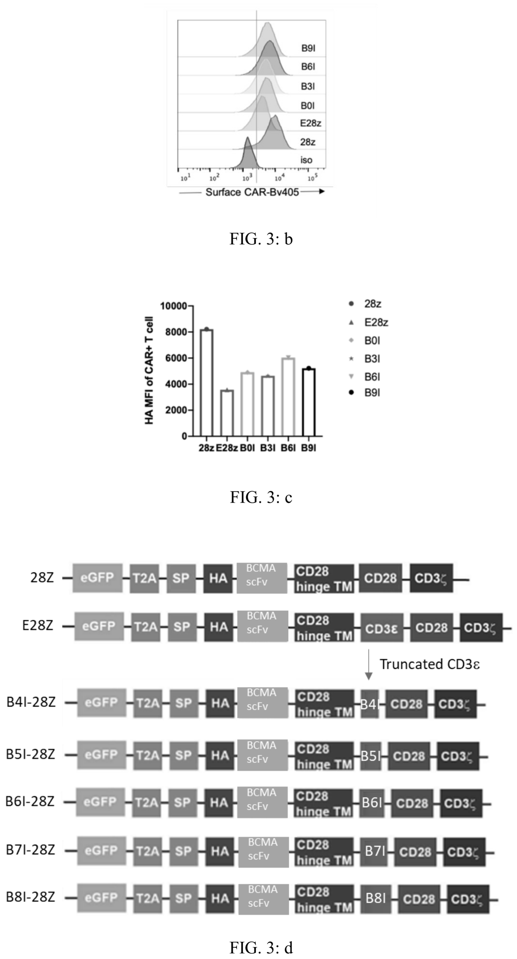

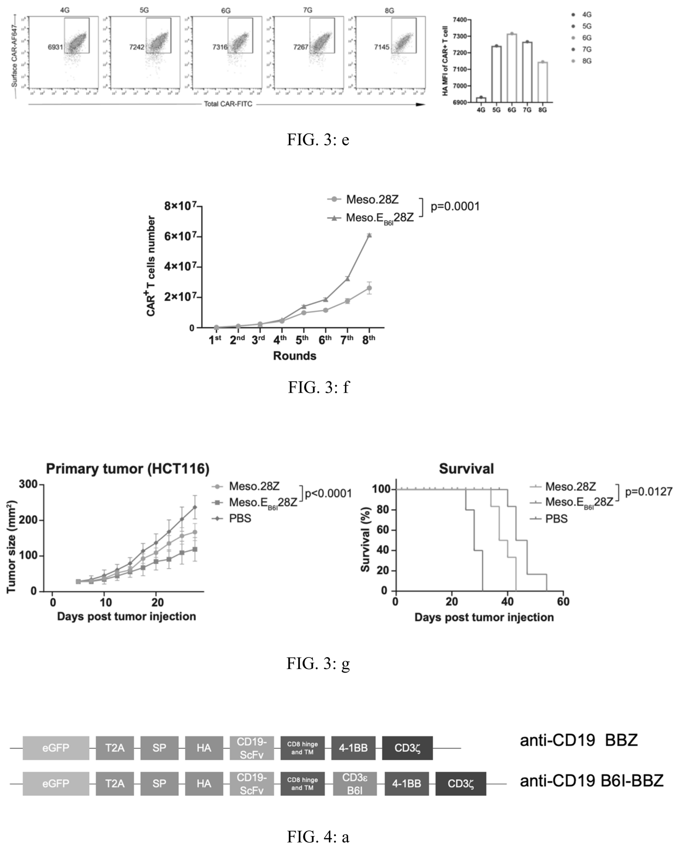

FIG. 3: a shows the structures of anti-mesothelin 28Z CAR, E28Z CAR, B0I-28Z CAR, B3I-28Z CAR, B6I-28Z CAR, and B9I-28Z CAR.
FIG. 3: b shows flow cytometry plots of surface expression levels of anti-mesothelin CAR on 28Z, E28Z, B0I-28Z, B3I-28Z, B6I-28Z, and B9I-28Z CAR-T cells.
FIG. 3: c shows statistical analysis of surface expression levels of anti-mesothelin CAR on 28Z, E28Z, B0I-28Z, B3I-28Z, B6I-28Z, and B9I-28Z CAR-T cells.
FIG. 3: d shows a schematic diagram of the structures of anti-BCMA 28Z CAR, E28Z CAR, B4I-28Z CAR, B5I-28Z CAR, B6I-28Z CAR, B7I-28Z CAR, and B8I-28Z CAR.
FIG. 3: e shows flow cytometry plots and statistical analysis of surface expression levels of anti-BCMA CAR on B4I-28Z, B5I-28Z, B6I-28Z, B7I-28Z, and B8I-28Z CAR-T cells.
FIG. 3: f shows growth curves of Anti-Mesothelin CAR-T cells under repeated tumor stimulation conditions.
FIG. 3: g shows the tumor control effect and survival curves under an HCT116 xenograft tumor model treated with Anti-Mesothelin CAR-T cells.

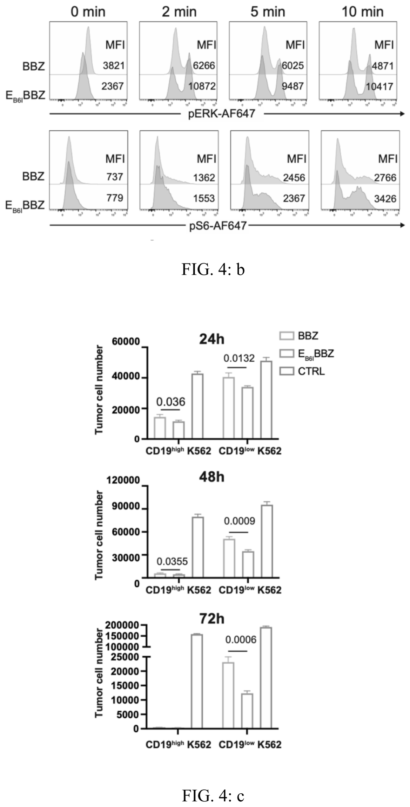

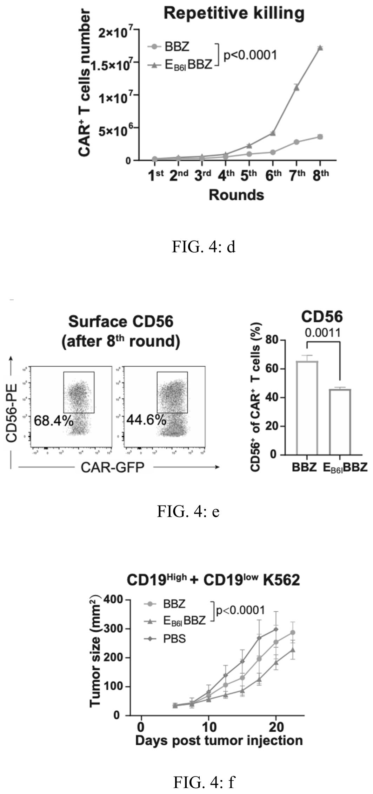

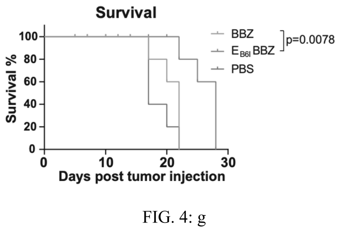

FIG. 4: a shows a schematic diagram of the structure of Anti-CD19 B6I-BBZ CAR.
FIG. 4: b shows flow cytometry plots of phosphorylated downstream signaling molecules ERK and S6 of CAR-T cells stimulated with low-CD19-expressing target cells.
FIG. 4: c shows the short-term tumor killing activity of BBZ and B6I-BBZ CAR-T cells.
FIG. 4: d shows the growth curve of Anti-CD19 BBZ and B6I-BBZ CAR-T cells under repeated tumor killing conditions.
FIG. 4: e shows CD56 expression levels in Anti-CD19 BBZ and B6I-BBZ CAR-T cells after the eighth round of repetitive killing.
FIG. 4: f shows the tumor control effect under the HCT116 xenograft tumor model treated with Anti-CD19 BBZ and B6I-BBZ CAR-T cells.
FIG. 4: g shows the survival curve under the HCT116 xenograft tumor model treated with Anti-CD19 BBZ and B6I-BBZ CAR-T cells.

### DETAILED DESCRIPTION

In one aspect of the present disclosure, a chimeric antigen receptor is provided, including:
an extracellular domain, a transmembrane domain, and an intracellular domain, which are sequentially connected;
wherein the extracellular domain includes an antigen-binding region and a hinge region; and
the intracellular domain includes, in sequence, a truncated CD3ε intracellular region, a costimulatory signaling region, and a CD3ζ intracellular region.

In the chimeric antigen receptor of the present disclosure, compared to the CD3ε intracellular region (see patent CN112500492A), the truncated CD3ε intracellular region consists of the basic residue rich sequence (BRS), a linker sequence, and the immunoreceptor tyrosine-based activation motif (ITAM) sequence linked in sequence, wherein the linker sequence is provided between the BRS sequence and the ITAM sequence.

In certain embodiments, the linker sequence may be a linker commonly used in the art for antibodies, such as a linker containing glycine (G) and serine (S). In some embodiments, the linker sequence may be a poly-glycine (poly-G) sequence, a poly-serine (poly-S) sequence, or a poly glycine-serine (Gly-Ser) sequence. In addition to glycine (G) and serine (S), the linker sequence may further include other known amino acid residues, such as alanine (A), leucine (L), threonine (T), glutamic acid (E), phenylalanine (F), arginine (R), glutamine (Q), etc. In one specific embodiment, the number of the amino acid residues in the linker sequence is in the range of 0-9. Specifically, it may be in the range of 0-3, 3-6, 6-9, etc.

In one specific embodiment, the truncated CD3ε intracellular region is engineered based on the CD3ε intracellular region sequence, retaining the BRS sequence and the ITAM sequence of the CD3ε intracellular region, and incorporating six glycine (G) residues as the linker sequence. The truncated CD3ε intracellular region is designated as B6I. Furthermore, the amino acid sequence of the truncated CD3ε intracellular region is as shown in SEQ ID NO: 1, specifically as follows:
KNRKAKAKGGGGGGDYEPIRKGQRDLYSGLNQRRI (B6I)

In the chimeric antigen receptor provided herein, the costimulatory signaling region is one or more of the intracellular regions of CD27, CD28, CD134, 4-1BB, and inducible co-stimulator (ICOS).

In one specific embodiment, the costimulatory signaling region is the intracellular region of CD28.

The amino acid sequence of the intracellular region of CD28 is as shown in SEQ ID NO: 2, specifically as follows:
RSKRSRLLHSDYMNMTPRRPGPTRKHYQPYAPPRDFAAYRS.

In another specific embodiment, the costimulatory signaling region is the intracellular region of 4-1BB.

The amino acid sequence of the intracellular region of 4-1BB is as shown in SEQ ID NO: 3, specifically as follows:
KRGRKKLLYIFKQPFMRPVQTTQEEDGCSCRFPEEEEGGCEL.

In the chimeric antigen receptor provided herein, the amino acid sequence of the CD3ζ intracellular region is as shown in SEQ ID NO: 4, specifically as follows:

RVKFSRSADAPAYQQGQNQLYNELNLGRREEYDVLDKRRGRDPEMGGKPRR
KNPQEGLYNELQKDKMAEAYSEIGMKGERRRGKGHDGLYQGLSTATKDTYDA
LHMQALPPR.

In the chimeric antigen receptor provided herein, the antigen-binding region is a single-chain fragment variable (scFv) targeting a tumor surface antigen.

In some embodiments, the single-chain fragment variable is one or more of CD19-, glypican-3- (GPC3), mesothelin-, CD20-, CD22-, CD123-, CD30-, CD33-, CD38-, CD138-, B cell maturation antigen- (BCMA), fibroblast activation protein- (FAP), carcinoembryonic antigen- (CEA), epidermal growth factor receptor variant III-(EGFRvIII), prostate-specific membrane antigen- (PSMA), human epidermal growth factor receptor 2- (Her2), interleukin 13 receptor alpha 2- (IL13Rα2), CD171-, and GD2-binding scFvs.

In certain embodiments, the single-chain fragment variable includes a light-chain variable region (VL) and a heavy-chain variable region (VH).

In some embodiments, the light-chain variable region and the heavy-chain variable region are linked via a linker sequence.

In one specific embodiment, the single-chain fragment variable is selected from CD19-, GPC3-, mesothelin-, and BCMA-binding scFvs. The monoclonal antibodies corresponding to CD19, GPC3, and mesothelin are FMC63, GC33, and SS1, respectively. The BCMA-binding monoclonal antibody is commercially available (e.g., from companies such as Shenzhen Pregene Biopharma Co., Ltd.). The sequences of the above antibodies are publicly known, and these antibodies possess high target specificity.

In one specific embodiment, the single-chain fragment variable includes the VL and VH of the FMC63 monoclonal antibody, which are optionally linked via a linker sequence.

In one specific embodiment, the single-chain fragment variable includes the VL and VH of the GC33 monoclonal antibody, which are optionally linked via a linker sequence.

In one specific embodiment, the single-chain fragment variable includes the VL and VH of the SS1 monoclonal antibody, which are optionally linked via a linker sequence.

The amino acid sequence of the FMC63 scFv is as shown in SEQ ID NO: 10, specifically as follows:

DIQMTQTTSSLSASLGDRVTISCRASQDISKYLNWYQQKPDGTVKLLIYHTSRL
HSGVPSRFSGSGSGTDYSLTISNLEQEDIATYFCQQGNTLPYTFGGGTKLEITGST
SGSGKPGSGEGSTKGEVKLQESGPGLVAPSQSLSVTCTVSGVSLPDYGVSWIRQP
PRKGLEWLGVIWGSETTYYNSALKSRLTIIKDNSKSQVFLKMNSLQTDDTAIYY
CAKHYYYGGSYAMDYWGQGTSVTVSS.

The amino acid sequence of the GC33 scFv is as shown in SEQ ID NO: 11, specifically as follows:

DVVMTQSPLSLPVTPGEPASISCRSSQSLVHSNANTYLHWYLQKPGQSPQLLIY
KVSNRFSGVPDRFSGSGSGTDFTLKISRVEAEDVGVYYCSQNTHVPPTFGQGTKL
EIKRGGGGSGGGGSGGGGSQVQLVQSGAEVKKPGASVKVSCKASGYTFTDYEM
HWVRQAPGQGLEWMGALDPKTGDTAYSQKFKGRVTLTADESTSTAYMELSSL
RSEDTAVYYCTRFYSYTYWGQGTLVTVSS.

The amino acid sequence of the SS1 scFv is as shown in SEQ ID NO: 20, specifically as follows:

QVQLQQSGPELEKPGASVKISCKASGYSFTGYTMNWVKQSHGKSLEWIGLITP
YNGASSYNQKFRGKATLTVDKSSSTAYMDLLSLTSEDSAVYFCARGGYDGRGF
DYWGQGTTVTVSSGGGGSGGGGSSGGGSDIELTQSPAIMSASPGEKVTMTCSAS
SSVSYMHWYQQKSGTSPKRWIYDTSKLASGVPGRFSGSGSGNSYSLTISSVEAE
DDATYYCQQWSKHPLTYGAGTKLEIK.

The amino acid sequence of the BCMA scFv may be found in patent CN109134665B.

Furthermore, the extracellular domain may additionally include a signal peptide, forming a structure in which the signal peptide, antigen-binding region, and hinge region are connected sequentially.

In the chimeric antigen receptor provided herein, the transmembrane domain is
CD28TM, CD4, CD8α, OX40, or H2-Kb.

In one specific embodiment, the transmembrane domain is CD28TM. The amino acid sequence of CD28TM is as shown in SEQ ID NO: 5, specifically:
FWVLVVVGGVLACYSLLVTVAFIIFWV.

Furthermore, the extracellular domain may additionally include a signal peptide, forming a structure in which the signal peptide, antigen-binding region, and hinge region are connected sequentially.

In certain embodiments, the amino acid sequence of the chimeric antigen receptor is as shown in SEQ ID NO: 6 (Anti-CD19 B6I-28Z CAR), SEQ ID NO: 7 (Anti-GPC3 B6I-28Z CAR), or SEQ ID NO: 21 (Anti-mesothelin B6I-28Z CAR).

SEQ ID NO: 6 (Anti-CD19 B6I-28Z CAR):
MVSKGEELFTGVVPILVELDGDVNGHKFSVSGEGEGDATYGKLTLKFICTTGK
LPVPWPTLVTTLTYGVQCFSRYPDHMKQHDFFKSAMPEGYVQERTIFFKDDGN
YKTRAEVKFEGDTLVNRIELKGIDFKEDGNILGHKLEYNYNSHNVYIMADKQKN
GIKVNFKIRHNIEDGSVQLADHYQQNTPIGDGPVLLPDNHYLSTQSALSKDPNEK
RDHMVLLEFVTAAGITLGMDELYKGSGEGRGSLLTCGDVEENPGPMLLLVTSLL
LCELPHPAFLLIPYPYDVPDYADIQMTQTTSSLSASLGDRVTISCRASQDISKYLN
WYQQKPDGTVKLLIYHTSRLHSGVPSRFSGSGSGTDYSLTISNLEQEDIATYFCQ
QGNTLPYTFGGGTKLEITGSTSGSGKPGSGEGSTKGEVKLQESGPGLVAPSQSLS
VTCTVSGVSLPDYGVSWIRQPPRKGLEWLGVIWGSETTYYNSALKSRLTIIKDNS
KSQVFLKMNSLQTDDTAIYYCAKHYYYGGSYAMDYWGQGTSVTVSSAAAIEV
MYPPPYLDNEKSNGTIIHVKGKHLCPSPLFPGPSKPFWVLVVVGGVLACYSLLVT
VAFIIFWVKNRKAKAKGGGGGGDYEPIRKGQRDLYSGLNQRRIRSKRSRLLHSD

YMNMTPRRPGPTRKHYQPYAPPRDFAAYRSRVKFSRSADAPAYQQGQNQLYNE
LNLGRREEYDVLDKRRGRDPEMGGKPRRKNPQEGLYNELQKDKMAEAYSEIG
MKGERRRGKGHDGLYQGLSTATKDTYDALHMQALPPR*.

The nucleotide sequence corresponding to SEQ ID NO: 6 (Anti-CD19 B6I-28Z CAR) is shown as SEQ ID NO: 8 (Anti-CD19 B6I-28Z CAR), and SEQ ID NO: 8 is shown as follows:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCgacatccagatgacacagactacatcctccctgtctgcctctctggga
gacagagtcaccatcagttgcagggcaagtcaggacattagtaaatatttaaattggtatcagcagaaaccagatggaactgtta
aactcctgatctaccatacatcaagattacactcaggagtcccatcaaggttcagtggcagtgggtctggaacagattattctctca
ccattagcaacctggagcaagaagatattgccacttacttttgccaacagggtaatacgcttccgtacacgttcggagggggga
ctaagttggaaataacaggctccacctctggatccggcaagcccggatctggcgagggatccaccaagggcgaggtgaaact
gcaggagtcaggacctggcctggtggcgccctcacagagcctgtccgtcacatgcactgtctcaggggtctcattacccgact
atggtgtaagctggattcgccagcctccacgaaagggtctggagtggctgggagtaatatggggtagtgaaaccacatactata
attcagctctcaaatccagactgaccatcatcaaggacaactccaagagccaagttttcttaaaaatgaacagtctgcaaactgat
gacacagccatttactactgtgccaaacattattactacggtggtagctatgctatggactactggggtcaaggaacctcagtcac
cgtctcctcagcggccgcaattgaagttatgtatcctcctccttacctagacaatgagaagagcaatggaaccattatccatgtga
aagggaaacacctttgtccaagtcccctatttcccggaccttctaagcccttttgggtgctggtggtggttgggggagtcctggctt
gctatagcttgctagtaacagtggcctttattattttctgggtgaagaatagaaaAgccaaggcTaaAggcggagggGGT
GGCGGAgactatgagcccatccggaaaggccagcgggacctgtattctggcctgaatcagagacgcatcaggagtaag
aggagcaggctcctgcacagtgactacatgaacatgactccccgccgccccgggcccacccgcaagcattaccagccctatg
ccccaccacgcgacttcgcagcctatcgctccagagtgaagttcagcaggagcgcagacgcccccgcgtaccagcagggcc
agaaccagctctataacgagctcaatctaggacgaagagaggagtacgatgttttggacaagagacgtggccgggaccctga
gatggggggaaagccgagaaggaagaaccctcaggaaggcctgtacaatgaactgcagaaagataagatggcggaggcct

acagtgagattgggatgaaaggcgagcgccggaggggcaaggggcacgatggcctttaccagggtctcagtacagccacc
aaggacacctacgacgcccttcacatgcaggccctgccccctcgctaa.

SEQ ID NO: 7 (Anti-GPC3 B6I-28Z CAR):

MVSKGEELFTGVVPILVELDGDVNGHKFSVSGEGEGDATYGKLTLKFICTTGK
LPVPWPTLVTTLTYGVQCFSRYPDHMKQHDFFKSAMPEGYVQERTIFFKDDGN
YKTRAEVKFEGDTLVNRIELKGIDFKEDGNILGHKLEYNYNSHNVYIMADKQKN
GIKVNFKIRHNIEDGSVQLADHYQQNTPIGDGPVLLPDNHYLSTQSALSKDPNEK
RDHMVLLEFVTAAGITLGMDELYKGSGEGRGSLLTCGDVEENPGPMLLLVTSLL
LCELPHPAFLLIPYPYDVPDYADVVMTQSPLSLPVTPGEPASISCRSSQSLVHSNA
NTYLHWYLQKPGQSPQLLIYKVSNRFSGVPDRFSGSGSGTDFTLKISRVEAEDVG
VYYCSQNTHVPPTFGQGTKLEIKRGGGGSGGGGSGGGGSQVQLVQSGAEVKKP
GASVKVSCKASGYTFTDYEMHWVRQAPGQGLEWMGALDPKTGDTAYSQKFK
GRVTLTADESTSTAYMELSSLRSEDTAVYYCTRFYSYTYWGQGTLVTVSSAAAI
EVMYPPPYLDNEKSNGTIIHVKGKHLCPSPLFPGPSKPFWVLVVVGGVLACYSLL
VTVAFIIFWVKNRKAKAKGGGGGGDYEPIRKGQRDLYSGLNQRRIRSKRSRLLH
SDYMNMTPRRPGPTRKHYQPYAPPRDFAAYRSRVKFSRSADAPAYQQGQNQLY
NELNLGRREEYDVLDKRRGRDPEMGGKPRRKNPQEGLYNELQKDKMAEAYSEI
GMKGERRRGKGHDGLYQGLSTATKDTYDALHMQALPPR*.

The nucleotide sequence corresponding to SEQ ID NO: 7 (Anti-GPC3 B6I-28Z CAR) is shown as SEQ ID NO: 9 (Anti-GPC3 B6I-28Z CAR), and SEQ ID NO: 9 is shown as follows:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCgatgttgtgatgactcagtctccactctccctgcccgtcacccctgga
gagccggcctccatctcctgcagatctagtcagagccttgtacacagtaatgccaacacctatttacattggtacctgcagaagc
cagggcagtctccacagctcctgatctataaagtttccaaccgattttctggggtccctgacaggttcagtggcagtggatcagg

cacagattttacactgaaaatcagcagagtggaggctgaggatgttggggtttattactgctctcaaaatacacatgttcctcctac
gtttggccaggggaccaagctggagatcaaacgtggtggaggcggttcaggcggaggtggctctggcggtggcggatcgca
ggtgcagctggtgcagtctggagctgaggtgaagaagcctggggcctcagtgaaggtctcctgcaaggcttctggatacacct
tcaccgactatgaaatgcactgggtgcgacaggcccctggacaagggcttgagtggatgggagctcttgatcctaaaactggt
gatactgcctacagtcagaagttcaagggcagagtcacgctgaccgcggacgaatccacgagcacagcctacatggagctga
gcagcctgagatctgaggacacggccgtgtattactgtacaagattctactcctatacttactggggccagggaaccctggtcac
cgtctcctcagcggccgcaattgaagttatgtatcctcctccttacctagacaatgagaagagcaatggaaccattatccatgtga
aagggaaacacctttgtccaagtcccctatttcccggaccttctaagcccttttgggtgctggtggtggttgggggagtcctggctt
gctatagcttgctagtaacagtggcctttattattttctgggtgaagaatagaaaAgccaaggcTaaAggcggagggGGT
GGCGGAgactatgagcccatccggaaaggccagcgggacctgtattctggcctgaatcagagacgcatcaggagtaag
aggagcaggctcctgcacagtgactacatgaacatgactccccgccgccccgggcccacccgcaagcattaccagccctatg
ccccaccacgcgacttcgcagcctatcgctccagagtgaagttcagcaggagcgcagacgcccccgcgtaccagcagggcc
agaaccagctctataacgagctcaatctaggacgaagagaggagtacgatgttttggacaagagacgtggccgggaccctga
gatggggggaaagccgagaaggaagaaccctcaggaaggcctgtacaatgaactgcagaaagataagatggcggaggcct
acagtgagattgggatgaaaggcgagcgccggaggggcaaggggcacgatggcctttaccagggtctcagtacagccacc
aaggacacctacgacgcccttcacatgcaggccctgccccctcgctaa.

SEQ ID NO: 21 (Anti-mesothelin B6I-28Z CAR):

MVSKGEELFTGVVPILVELDGDVNGHKFSVSGEGEGDATYGKLTLKFICTTGK
LPVPWPTLVTTLTYGVQCFSRYPDHMKQHDFFKSAMPEGYVQERTIFFKDDGN
YKTRAEVKFEGDTLVNRIELKGIDFKEDGNILGHKLEYNYNSHNVYIMADKQKN
GIKVNFKIRHNIEDGSVQLADHYQQNTPIGDGPVLLPDNHYLSTQSALSKDPNEK
RDHMVLLEFVTAAGITLGMDELYKGSGEGRGSLLTCGDVEENPGPMLLLVTSLL
LCELPHPAFLLIPYPYDVPDYAQVQLQQSGPELEKPGASVKISCKASGYSFTGYT
MNWVKQSHGKSLEWIGLITPYNGASSYNQKFRGKATLTVDKSSSTAYMDLLSL
TSEDSAVYFCARGGYDGRGFDYWGQGTTVTVSSGGGGSGGGGSSGGGSDIELT
QSPAIMSASPGEKVTMTCSASSSVSYMHWYQQKSGTSPKRWIYDTSKLASGVPG
RFSGSGSGNSYSLTISSVEAEDDATYYCQQWSKHPLTYGAGTKLEIKAAAIEVM
YPPPYLDNEKSNGTIIHVKGKHLCPSPLFPGPSKPFWVLVVVGGVLACYSLLVTV
AFIIFWVKNRKAKAKGGGGGGDYEPIRKGQRDLYSGLNQRRIRSKRSRLLHSDY
MNMTPRRPGPTRKHYQPYAPPRDFAAYRSRVKFSRSADAPAYQQGQNQLYNEL
NLGRREEYDVLDKRRGRDPEMGGKPRRKNPQEGLYNELQKDKMAEAYSEIGM
KGERRRGKGHDGLYQGLSTATKDTYDALHMQALPPR*.

The nucleotide sequence corresponding to SEQ ID NO: 21 (Anti-mesothelin B6I-28Z CAR) is shown as SEQ ID NO: 19 (Anti-mesothelin B6I-28Z CAR), and SEQ ID NO: 19 is shown as follows:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA

GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCCAGGTACAACTGCAGCAGTCTGGGCCTGA
GCTGGAGAAGCCTGGCGCTTCAGTGAAGATATCCTGCAAGGCTTCTGGTTAC
TCATTCACTGGCTACACCATGAACTGGGTGAAGCAGAGCCATGGAAAGAGC
CTTGAGTGGATTGGACTTATTACTCCTTACAATGGTGCTTCTAGCTACAACCA
GAAGTTCAGGGGCAAGGCCACATTAACTGTAGACAAGTCATCCAGCACAGC
CTACATGGACCTCCTCAGTCTGACATCTGAAGACTCTGCAGTCTATTTCTGTG
CAAGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGCGG
TGGTGGATCGGACATCGAGCTCACTCAGTCTCCAGCAATCATGTCTGCATCT
CCAGGGGAGAAGGTCACCATGACCTGCAGTGCCAGCTCAAGTGTAAGTTAC
ATGCACTGGTACCAGCAGAAGTCAGGCACCTCCCCCAAAAGATGGATTTATG
ACACATCCAAACTGGCTTCTGGAGTCCCAGGTCGCTTCAGTGGCAGTGGGTC
TGGAAACTCTTACTCTCTCACAATCAGCAGCGTGGAGGCTGAAGATGATGCA
ACTTATTACTGCCAGCAGTGGAGTAAGCACCCTCTCACGTACGGTGCTGGGA
CAAAGTTGGAAATCAAAgcggccgcaattgaagttatgtatcctcctccttacctagacaatgagaagagcaat
ggaaccattatccatgtgaaagggaaacacctttgtccaagtcccctatttcccggaccttctaagcccttttgggtgctggtggtg
gttgggggagtcctggcttgctatagcttgctagtaacagtggcctttattattttctgggtgaagaatagaaaAgccaaggcTa
aAggcggagggGGTGGCGGAgactatgagcccatccggaaaggccagcgggacctgtattctggcctgaatcaga
gacgcatcaggagtaagaggagcaggctcctgcacagtgactacatgaacatgactccccgccgccccgggcccacccgca
agcattaccagccctatgccccaccacgcgacttcgcagcctatcgctccagagtgaagttcagcaggagcgcagacgcccc
cgcgtaccagcagggccagaaccagctctataacgagctcaatctaggacgaagagaggagtacgatgttttggacaagaga
cgtggccgggaccctgagatggggggaaagccgagaaggaagaaccctcaggaaggcctgtacaatgaactgcagaaag
ataagatggcggaggcctacagtgagattgggatgaaaggcgagcgccggaggggcaaggggcacgatggcctttaccag
ggtctcagtacagccaccaaggacacctacgacgcccttcacatgcaggccctgccccctcgc.

The components forming the chimeric antigen receptor of the present disclosure may be directly connected to one another or may be linked through a linker sequence. The linker sequence may be a linker commonly used in the art for antibodies, such as a linker containing glycine (G) and serine (S). Typically, a linker includes one or more repeated motifs. Preferably, the motifs are arranged repeatedly within the linker sequence without intervening amino acid residues. The linker sequence may include 1, 2, 3, 4, or 5 repeated motifs. The linker may include 3 to 25 amino acid residues, for example, 3 to 15, 5 to 15, or 10 to 20 residues. In certain embodiments, the linker sequence is a polyglycine linker sequence. The number of glycine residues in the linker sequence is not particularly limited, and is typically in the range of 2-20, such as 2-15, 2-10, or 2-8. In addition to glycine (G) and serine (S), the linker sequence may further include other known amino acid residues, such as alanine (A), leucine (L), threonine (T), glutamic acid (E), phenylalanine (F), arginine (R), glutamine (Q), etc.

It should be understood that, during gene cloning operations, suitable restriction enzyme recognition sites are often engineered into the construct, which may introduce one or more extraneous residues at the terminus of the expressed amino acid sequence. However, such additions do not affect the activity of the intended sequence. Moreover, to construct fusion proteins, facilitate recombinant protein expression, obtain recombinant proteins that are autonomously secreted from host cells, or promote purification of recombinant proteins, certain amino acids may be added to the N-terminus, the C-terminus, or other appropriate positions within the recombinant protein. Such added sequences may include, but are not limited to, suitable linker peptides, signal peptides, leader peptides, terminal extensions, etc. Accordingly, the fusion protein (i.e., the CAR) of the present disclosure may further include one or more polypeptide fragments at the amino terminus or carboxyl terminus as protein tags. Any suitable tag may be used herein. Examples of tags include FLAG, HA, HA1, c-Myc, poly-His, poly-Arg, Strep-Tag II, AU1, EE, T7, 4A6, ε, B, gE, and Tyl. These tags may be used for the purification of the protein.

In another aspect of the present disclosure, a polynucleotide sequence is provided. The polynucleotide sequence includes:
(1) a polynucleotide sequence encoding the foregoing chimeric antigen receptor; and/or
(2) the complementary sequence of the polynucleotide sequence described in (1).

The polynucleotide sequence of the present disclosure may be in the form of DNA or RNA. The DNA form may include cDNA, genomic DNA, and synthetically produced DNA. The DNA may be single-stranded or double-stranded, and may be a coding strand or a non-coding strand. The present disclosure also encompasses degenerate variants of the polynucleotide sequence encoding the fusion protein; that is, polynucleotide sequences that differ in nucleotide sequence but encode the same amino acid sequence.

The polynucleotide sequences described herein may generally be obtained by PCR amplification. In particular, primers may be designed based on the nucleotide sequences disclosed herein, especially the open reading frames. Amplification may be carried out using commercially available cDNA libraries or cDNA libraries prepared by conventional methods known to those skilled in the art. When the sequence is relatively long, two or more rounds of PCR amplification may be required, and the resulting amplified fragments may then be assembled in the correct order.

In another aspect, the present disclosure provides a nucleic acid construct including the above-described polynucleotide sequence.

The nucleic acid construct further includes one or more regulatory sequences operably connected to the polynucleotide sequence. The coding sequence of the CAR provided herein may be manipulated in various ways to ensure protein expression. Prior to insertion of the nucleic acid construct into a vector, the construct may be modified depending on the type or requirements of the expression vector. Techniques for modifying polynucleotide sequences using recombinant DNA methods are well known in the art.

The regulatory sequence may be a suitable promoter sequence. The promoter sequence is usually operatively connected to the coding sequence of the protein to be expressed. The promoter may be any nucleotide sequence that exhibits transcriptional activity in the selected host cell, including mutated, truncated, and hybrid promoters, and may be derived from genes encoding extracellular or intracellular polypeptides that are homologous or heterologous to the host cell.

The regulatory sequence may also be a suitable transcription terminator sequence, recognized by the host cell to terminate transcription. The terminator sequence may be operably connected to the 3' terminus of the polynucleotide sequence encoding the polypeptide. Any terminator that is functional in the selected host cell may be used in the present disclosure.

The regulatory sequence may further be a suitable leader sequence, i.e., an untranslated region (UTR) of the mRNA that is important for translation in the host cell. The leader sequence may be operably connected to the 5' terminus of the polynucleotide sequence encoding the polypeptide. Any leader sequence that is functional in the selected host cell may be used in the present disclosure.

In a preferred embodiment of the present disclosure, the nucleic acid construct is a vector.

Expression of the polynucleotide sequence encoding the CAR is typically achieved by operably connecting the polynucleotide sequence encoding the CAR to a promoter and incorporating the resulting construct into an expression vector. The vector may be suitable for replication and integration in a eukaryotic cell. Typical cloning vectors contain transcription and translation terminators, initiation sequences, and promoters useful for regulating expression of the desired nucleic acid sequence.

The polynucleotide sequence encoding the CAR of the present disclosure may be cloned into numerous types of vectors. Examples include plasmids, phagemids, bacteriophage derivatives, animal viruses, and cosmids. Furthermore, the vector is an expression vector. The expression vector may be delivered to a cell in the form of a viral vector. Viral vector technologies are well known in the art. The viruses that may be used as vectors include, but are not limited to, retroviruses, adenoviruses, adeno-associated viruses, herpesviruses, and lentiviruses. Generally, a suitable vector may contain an origin of replication that functions in at least one organism, a promoter sequence, a convenient restriction enzyme recognition site, and one or more selectable markers.

In a specific embodiment of the present disclosure, the nucleic acid construct is a lentiviral vector, including an origin of replication, a 3' long terminal repeat (LTR), a 5' LTR, and the aforementioned polynucleotide sequence.

An example of a suitable promoter is the immediate early cytomegalovirus (CMV) promoter. This promoter is a strong constitutive promoter capable of driving high-level expression of any polynucleotide sequence operably linked thereto. Another example of a suitable promoter is the elongation factor-1α (EF-1α) promoter. However, other constitutive promoters may also be employed, including but not limited to the simian virus 40 (SV40) early promoter, mouse mammary tumor virus (MMTV) promoter, human immunodeficiency virus (HIV) long terminal repeat (LTR) promoter, MoMuLV promoter, avian leukosis virus promoter, Epstein-Barr virus immediate early promoter, Rous sarcoma virus promoter, and human gene promoters such as, but not limited to, actin promoter, myosin promoter, hemoglobin promoter, and creatine kinase promoter. Furthermore, inducible promoters may also be used. The use of an inducible promoter provides a molecular switch that can activate expression of the polynucleotide sequence operably linked to the inducible promoter at desired times and deactivate expression when expression is not desired. Examples of inducible promoters include, but are not limited to, the metallothionein promoter, glucocorticoid promoter, progesterone promoter, and tetracycline promoter.

To evaluate expression of the CAR or portions thereof, the expression vector introduced into the cell may further contain one or both of a selectable marker gene and a reporter gene, facilitating identification and selection of expressing cells from the population of cells transfected or infected by the viral vector. Alternatively, the selectable marker may be carried on a separate DNA fragment and used in a co-transfection procedure. Both selectable marker gene and reporter gene may be flanked by suitable regulatory sequences enabling expression in the host cell. Useful selectable marker genes include, for example, antibiotic resistance genes, such as neo.

Reporter genes are used to identify potentially transfected cells and to evaluate the functionality of regulatory sequences. After the DNA has been introduced into the recipient cells, expression of the reporter gene may be assessed at an appropriate time. Suitable reporter genes may include genes encoding luciferase, β-galactosidase, chloramphenicol acetyltransferase, secreted alkaline phosphatase, and green fluorescent protein. Appropriate expression systems are well known and may be prepared using known techniques or obtained commercially.

Methods for introducing genes into cells and expressing genes in cells are known in the art. Vectors may be readily introduced into host cells by any method known in the art, such as those used for mammalian, bacterial, yeast, or insect cells. For example, an expression vector may be transferred into a host cell by physical, chemical, or biological methods.

Physical methods for introducing polynucleotides into host cells include calcium phosphate precipitation, lipid transfection, particle bombardment, microinjection, electroporation, etc. Biological methods for introducing polynucleotides into host cells include the use of DNA and RNA vectors. Chemical methods for introducing polynucleotides into host cells include colloidal dispersion systems such as macromolecular complexes, nanocapsules, microspheres, beads, and lipid-based systems including water-in-oil emulsions, micelles, mixed micelles, and liposomes.

Biological methods for introducing polynucleotides into host cells include the use of viral vectors, particularly lentiviral vectors, which have become the most widely used methods for inserting genes into mammalian cells, including human cells. Other viral vectors may be derived from lentiviruses, poxviruses, herpes simplex virus type I, adenoviruses, and adeno-associated viruses. Various viral-based systems have been developed for gene delivery into mammalian cells. For example, lentiviruses provide a convenient platform for gene delivery systems. A selected gene may be inserted into a vector and packaged into lentiviral particles using known techniques. The recombinant virus may then be isolated and delivered to target cells *in vivo* or *ex vivo.* Numerous retroviral systems are known in the art. In some embodiments, an adenoviral vector is used. Many adenoviral vectors are well known in the art. In one embodiment, a lentiviral vector is used.

In another aspect, the present disclosure provides a lentiviral vector system, including the aforementioned nucleic acid construct and a lentiviral auxiliary component.

The lentiviral auxiliary component includes a lentiviral packaging plasmid and a cell line. The lentiviral vector system is formed by packaging the aforementioned nucleic acid construct with the aid of the lentiviral packaging plasmid and cell line. Methods for constructing lentiviral vector systems are commonly used methods in the field.

In another aspect, the present disclosure provides a method for *ex vivo* activation of T cells, including infecting the aforementioned T cells with the lentivirus.

The CAR-T cells of the present disclosure are capable of undergoing robust *in vivo* T cell expansion and persisting at high levels for extended periods in the blood and bone marrow, and forming antigen-specific memory T cells. Upon encountering and subsequently eliminating target cells expressing antigens, the CAR-T cells of the present disclosure may differentiate *in vivo* into a central memory-like phenotype.

The present application further provides a cellular therapy, in which T cells are genetically modified to express the CAR described herein, and CAR-T cells are administered to a subject in need thereof. The administered cells are capable of killing tumor cells in the subject. Unlike the antibody-based therapy, CAR-T cells are able to proliferate *in vivo,* thereby providing long-term persistence that can result in durable tumor control.

The antitumor immune response elicited by CAR-T cells may involve active or passive immune mechanisms. Additionally, CAR-mediated immune responses may form part of an adoptive immunotherapy, where CAR-T cells induce a specific immune response through the antigen-binding region of the CAR.

The cancers that may be treated include non-solid tumors, such as hematologic malignancies (e.g., leukemia and lymphoma). In particular, the CARs of the present disclosure, their encoding sequences, nucleic acid constructs, expression vectors, viral vectors, and CAR-T cells can be used to treat these diseases. The diseases may specifically include CD19- or GPC3-mediated diseases, especially CD19- or GPC3-mediated tumors.

Specifically, as used herein, "CD19-mediated diseases" include, but are not limited to, leukemia and lymphoma, such as B-cell lymphoma, mantle cell lymphoma, acute lymphoblastic leukemia, chronic lymphocytic leukemia, hairy cell leukemia, and acute myeloid leukemia.

Specifically, as used herein, glypican-3 (GPC3) is an extracellular matrix protein that is expressed in embryonic tissues, particularly in the liver and kidney, and is associated with organogenesis. "GPC3-mediated diseases" include, but are not limited to, hepatocellular carcinoma, melanoma, ovarian clear cell carcinoma, and lung squamous cell carcinoma, etc.

In another aspect, the present disclosure provides a genetically modified T cell, including the aforementioned polynucleotide sequence, or the aforementioned nucleic acid construct, or being infected with the aforementioned lentiviral vector system.

The CAR-modified T cells of the present disclosure may be administered alone or as a pharmaceutical composition in combination with a diluent and/or other components such as relevant cytokines or cell populations. In brief, the pharmaceutical composition of the present disclosure may include the CAR-T cells described herein in combination with one or more pharmaceutically or physiologically acceptable carriers, diluents, or excipients. Such compositions may include buffers such as neutral buffered saline or sulfate buffered saline; carbohydrates such as glucose, mannose, sucrose, dextran, or mannitol; proteins; polypeptides or amino acids such as glycine; antioxidants; chelating agents such as ethylenediaminetetraacetic acid (EDTA) or glutathione; adjuvants (e.g., aluminum hydroxide); and preservatives.

The pharmaceutical composition of the present disclosure may be administered in a manner suitable for treating (or preventing) the disease. The amount and frequency of administration will be determined by factors such as the patient's condition and the type and severity of the disease.

As used herein, the terms "immunologically effective amount," "antitumor effective amount," "tumor-inhibiting effective amount," "therapeutically effective amount," or "therapeutic amount" refer to an amount of the composition of the present disclosure that is determined by a clinician based on individual differences such as the patient's (subject's) age, weight, tumor size, degree of infection or metastasis, and the condition. In general, the pharmaceutical composition including the T cells described herein may be administered at a dose of 10⁴ to 10⁹ cells/kg body weight, preferably 10⁵ to 10⁶ cells/kg body weight. The composition containing the T cell may also be administered multiple times at these doses. The cells may be administered by injection techniques known in immunotherapy. Optimal dose/amount and treatment regimens for a particular patient can readily be determined by practitioners in the medical field by monitoring clinical indicators of disease, which facilitates the adjustment of the treatment.

Administration of the composition to the subject may be carried out in any convenient manner, including spray, injection, swallowing, infusion, implantation, or transplantation. The composition described herein may be administered subcutaneously, intracutaneously, intratumorally, intranodally, intraspinally, intramuscularly, intravenously, or intraperitoneally. In one embodiment, the composition containing the T cell of the present disclosure is administered to patients through intracutaneous or subcutaneous injection. In another embodiment, the composition containing the T cell is preferably administered via intravenous injection. The composition containing the T cell may also be directly injected into the tumor, lymph node, or the site of infection.

In certain embodiments of the present disclosure, the CAR-T cells or their compositions may be combined with other therapies known in the art. These therapies include, but are not limited to, chemotherapy, radiotherapy, and immunosuppressants. For example, the CAR-T cells or the composition thereof of the present disclosure may be combined with various radiotherapy agents, including cyclosporine, azathioprine, methotrexate, mycophenolate mofetil, FK506, fludarabine, rapamycin, and mycophenolic acid, etc. In a further embodiment, the composition of the present disclosure may be administered to a patient in combination with bone marrow transplantation, chemotherapeutic agents (e.g., before, concurrently with, or after) such as fludarabine, external beam radiation therapy (XRT), cyclophosphamide, or T cell ablation therapies utilizing antibodies such as OKT3 or CAMPATH.

As used herein, the term "antitumor effect" refers to a biological effect manifested by tumor volume reduction, reduced tumor cell number, decreased metastasis, prolonged survival, or improvement of various physiological symptoms associated with cancer.

The terms "patient," "subject," "individual," and "object," etc., are used interchangeably herein, referring to a living organism capable of mounting an immune response, such as a mammal or non-mammalian organism. The mammal may preferably be a rodent, an artiodactyl, a perissodactyl, a lagomorph, or a primate. The non-mammalian organism may include non-mammalian vertebrates such as birds (e.g., chickens or ducks), fish, and non-mammalian invertebrates. Examples include, but are not limited to, humans, dogs, cats, mice, rats, and their transgenic variants. In a specific embodiment, the subject is a human, for example, a patient who is immunocompromised or suffering from cancer.

The present disclosure provides a use of the foregoing chimeric antigen receptor, or the foregoing polynucleotide sequence, or the foregoing nucleic acid construct, or the foregoing lentiviral vector system, in the preparation of a product for one or more of the following purposes: (1) preparing CAR-T cells; (2) increasing surface expression level of CAR on CAR-T cells; (3) enhancing antigen sensitivity of CAR-T cells; (4) suppressing IFN-γ, IL-2, and TNF cytokine secretion of CAR-T cells; (5) promoting natural growth of CAR-T cells under resting conditions; (6) enhancing sustained proliferation of CAR-T cells after stimulation by target cells; (7) improving repetitive killing capacity of CAR-T cells; (8) reducing NK-like differentiation and exhaustion of CAR-T cells; (9) increasing stemness and memory cell subsets of CAR-T cells.

The present disclosure provides a use of the foregoing chimeric antigen receptor, or the foregoing polynucleotide sequence, or the foregoing nucleic acid construct, or the foregoing lentiviral vector system, or the foregoing genetically modified T cell, in the preparation of a product for tumor therapy.

In a specific embodiment of the present disclosure, the tumor is leukemia or a solid tumor.

In a preferred embodiment of the present disclosure, the tumor is B-cell lymphoma, mantle cell lymphoma, acute lymphoblastic leukemia, chronic lymphocytic leukemia, hairy cell leukemia, acute myeloid leukemia, hepatocellular carcinoma, melanoma, ovarian clear cell carcinoma, or lung squamous cell carcinoma.

The present disclosure also provides a method for treating a tumor, including administering to a subject in need thereof a therapeutically effective amount of the genetically modified T cell.

The terms "treatment" or "therapy" of a condition include preventing or alleviating the condition, slowing the onset or progression of the condition, reducing the risk of developing the condition, preventing or delaying the development of symptoms associated with the condition, reducing or eliminating symptoms associated with the condition, partially or completely reversing the condition, curing the condition, or any combination thereof. For cancer, "treatment" or "therapy" may refer to inhibiting or slowing the growth, proliferation, or metastasis of tumors or malignant cells, or any combination thereof. For tumors, "treatment" or "therapy" includes eliminating all or part of the tumor, inhibiting or slowing tumor growth and metastasis, preventing or delaying tumor progression, or any combination thereof.

When performing administration on a subject, the dose/amount may vary based on the patient's age and weight, the characteristics and severity of the disease, as well as the route of administration. The dose/amount can be adjusted based on the results of animal experiments and other relevant factors, and the total administered dose should not exceed a certain limit.

The present disclosure is further illustrated through specific examples; those skilled in the art can easily understand other advantages and effects of the present disclosure based on the content disclosed in this specification. The present disclosure may also be implemented or applied through various other specific embodiments. The details disclosed herein may be modified or altered from different perspectives or for different applications without departing from the spirit of the present disclosure.

Before further describing the specific embodiments of the present disclosure, it should be understood that the scope of the present disclosure is not limited to the specific examples provided below. It should also be understood that the term used in the examples is intended to describe particular embodiments and not to limit the scope of the present disclosure. Unless otherwise explicitly stated, the singular forms "a", "an", and "the" include the plural forms.

When numerical ranges are provided in the examples, it should be understood that any value within the range, including the endpoints, may be selected, unless otherwise specified. Unless otherwise defined, all technical and scientific terms used herein have the same meaning as commonly understood by those skilled in the art. Apart from the specific methods, devices, and materials described in the examples, those skilled in the art may use any similar or equivalent methods, devices, and materials known in the prior art to achieve the same results as described in the embodiments of the present disclosure.

Unless otherwise stated, the experimental methods, detection methods, and preparation methods disclosed in the present disclosure all employ conventional techniques in the technical field of molecular biology, biochemistry, chromatin structure and analysis, analytical chemistry, cell culture, recombinant DNA technology and other related fields.

### Example 1

Anti-CD19 B6I-28Z was generated based on the anti-CD19 E28Z sequence (see patent CN112500492A). The BRS sequence and the ITAM sequence of the CD3ε intracellular region were retained, and six glycine residues (6×G) were used as a linker sequence to construct a truncated CD3ε intracellular region. The resulting chimeric antigen receptor was designated as B6I-28Z.

The nucleotide sequence of the Anti-CD19 B6I-28Z CAR is as shown in SEQ ID NO: 8. Specifically:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC

CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCgacatccagatgacacagactacatcctccctgtctgcctctctggga
gacagagtcaccatcagttgcagggcaagtcaggacattagtaaatatttaaattggtatcagcagaaaccagatggaactgtta
aactcctgatctaccatacatcaagattacactcaggagtcccatcaaggttcagtggcagtgggtctggaacagattattctctca
ccattagcaacctggagcaagaagatattgccacttacttttgccaacagggtaatacgcttccgtacacgttcggagggggga
ctaagttggaaataacaggctccacctctggatccggcaagcccggatctggcgagggatccaccaagggcgaggtgaaact
gcaggagtcaggacctggcctggtggcgccctcacagagcctgtccgtcacatgcactgtctcaggggtctcattacccgact
atggtgtaagctggattcgccagcctccacgaaagggtctggagtggctgggagtaatatggggtagtgaaaccacatactata
attcagctctcaaatccagactgaccatcatcaaggacaactccaagagccaagttttcttaaaaatgaacagtctgcaaactgat
gacacagccatttactactgtgccaaacattattactacggtggtagctatgctatggactactggggtcaaggaacctcagtcac
cgtctcctcagcggccgcaattgaagttatgtatcctcctccttacctagacaatgagaagagcaatggaaccattatccatgtga
aagggaaacacctttgtccaagtcccctatttcccggaccttctaagcccttttgggtgctggtggtggttgggggagtcctggctt
gctatagcttgctagtaacagtggcctttattattttctgggtgaagaatagaaaAgccaaggcTaaAggcggagggGGT
GGCGGAgactatgagcccatccggaaaggccagcgggacctgtattctggcctgaatcagagacgcatcaggagtaag
aggagcaggctcctgcacagtgactacatgaacatgactccccgccgccccgggcccacccgcaagcattaccagccctatg
ccccaccacgcgacttcgcagcctatcgctccagagtgaagttcagcaggagcgcagacgcccccgcgtaccagcagggcc
agaaccagctctataacgagctcaatctaggacgaagagaggagtacgatgttttggacaagagacgtggccgggaccctga
gatggggggaaagccgagaaggaagaaccctcaggaaggcctgtacaatgaactgcagaaagataagatggcggaggcct
acagtgagattgggatgaaaggcgagcgccggaggggcaaggggcacgatggcctttaccagggtctcagtacagccacc
aaggacacctacgacgcccttcacatgcaggccctgccccctcgctaa.

The Anti-CD19 B6I-28Z CAR was subcloned into the lentiviral expression plasmid pHAGE vector. The CAR expression plasmid, packaging plasmid pSPAX2, and pMD2G were mixed at a ratio of 10:7.5:3.5 and transfected into 293FT cells using the calcium phosphate transfection method to produce lentiviral particles. Using ultracentrifugation, lentiviruses expressing 28Z CAR, E28Z CAR (see patent CN112500492A), and B6I-28Z CAR were concentrated into small-volume, high-titer viral stocks (~10⁸/ml). These viruses were then used to transduce primary T cells activated for one day with αCD3/αCD28 Ab-beads (MOI = 10). The primary T cells were cultured in a complete T-cell medium (XIVO-15 + 1% P.S. + 10 ng/ml human IL-7 + 10 ng/ml human IL-15 + 5% Human AB serum + 10 mM neutralized NAC). The viruses were removed one day after transduction, and the antibody was removed four days after antibody stimulation. On Day 5 post-stimulation, CAR-T cells (GFP⁺) were sorted. After a period of expansion, CAR-T cells were characterized by a series of indicators, including CAR surface expression levels, CD4/CD8 subset ratio, *in vitro* cytokine secretion, *in vitro* killing ability, *in vitro* proliferation ability, *in vitro* apoptosis level, *in vitro* sustained proliferative capacity, and *in vivo* antitumor efficacy.

The structures of anti-CD19 28Z CAR, E28Z CAR, and B6I-28Z CAR are shown in FIG. 1: a.

Surface expression levels of CAR molecules on 28Z CAR-T, E28Z CAR-T, and B6I-28Z CAR-T cells were detected by flow cytometry using anti-HA rabbit primary antibody and anti-rabbit AF647 secondary antibody. The flow cytometry results, shown in FIG. 1: b, indicate that B6I-28Z CAR-T cells restored the reduced surface CAR expression observed in E28Z CAR-T cells.

28Z CAR-T, E28Z CAR-T, and B6I-28Z CAR-T cells were detected by flow cytometry using anti-human CD4-PB and anti-human CD8-PE antibodies. The flow cytometry results, shown in FIG. 1: c, demonstrate that the ratios of CD4⁺ CAR-T cells to CD8⁺ CAR-T cells were similar among the three groups.

Memory subset distribution of 28Z CAR-T, E28Z CAR-T, and B6I-28Z CAR-T cells was analyzed by flow cytometry using anti-human CD62L-APC and anti-human CD45RA-PE-Cy7 antibodies. The flow cytometry results, shown in FIG. 1: d, demonstrate that B6I-28Z CAR-T cells exhibited the highest proportion of stem cell-like central memory cell subsets (CD62L⁺CD45RA⁺), followed by E28Z CAR-T cells, with 28Z CAR-T cells showing the lowest proportion.

When performing stimulation using 10 nM CD19 antigen together with the adhesion molecule ICAM-1, the immunological synapses formed by 28Z CAR-T, E28Z CAR-T, and B6I-28Z CAR-T cells are shown in FIG. 1: e. B6I-28Z CAR molecules displayed stronger fluorescence intensity and showed more efficient recruitment of the downstream signaling molecule Zap70.

Raji cells, low-antigen-expressing K562 cells (K562 L), very-low-antigen-expressing K562 cells (K562 VL), and CAR-T cells were co-incubated. At 10 minutes and 30 minutes, the percentage of target cells bound to CAR-T cells was measured. The results, shown in FIG. 1: f, indicate that B6I-28Z CAR-T cells and E28Z CAR-T cells exhibited stronger cell-cell contact ability than 28Z CAR-T cells, demonstrating that B6I CAR-T cells possess enhanced antigen sensitivity.

To simulate the scenario of antigen downregulation during tumor immune escape, K562 H, K562 L, and CAR-T cells were mixed at a ratio of 1:1:2. After 30 hours of incubation, the remaining numbers of K562 H and K562 L cells were measured. As shown in FIG. 1: g, B6I-28Z CAR-T cells achieved fewer surviving K562 L cells compared with 28Z CAR-T and E28Z CAR-T cells, further indicating superior antigen sensitivity.

Raji, K562 H, K562 L, and CAR-T cells were co-cultured at a ratio of 1:1. After 24 hours of incubation, the remaining Raji, K562 H, and K562 L cells were stained with anti-CD19-PE antibody to determine the mean fluorescence intensity (MFI) of surface CD19. As shown in FIG. 1: h, compared to 28Z CAR-T and E28Z CAR-T cells, the remaining target cells in the B6I-28Z CAR-T cell group showed lower surface antigen fluorescence intensity, indicating better antigen sensitivity.

1×10⁵ CAR-T cells and 1×10⁵ CD19⁺ Raji lymphoma cells were seeded at a ratio of 1:1 into a round-bottom 96-well plate, mixed thoroughly, centrifuged at room temperature (400g, 1 min) to promote cell-cell contact, and cultured in complete medium at 37°C for one day. Six hours prior to sample collection, 1× BFA was added to inhibit the secretion of cytokine. Afterwards, the collected cell samples were washed once with PBS, fixed with 4% PFA at room temperature for 5 min, permeabilized with 0.1% Triton X-100 at room temperature for 5 min, and subsequently subjected to standard intracellular staining to detect IL-2, IFN-γ, and TNF-α. The results, shown in FIG. 1: i, indicate that E28Z CAR-T and B6I-28Z CAR-T cells produced markedly lower levels of IL-2, IFN-γ, and TNF-α upon CD19⁺ cell-specific stimulation compared with 28Z CAR-T cells.

CAR-T cells were cultured without antigen stimulation for an extended period, and cell numbers were counted every 3-4 days. As shown in FIG. 1: j, B6I-28Z CAR-T cells exhibited better growth capacity than E28Z CAR-T and 28Z CAR-T cells.

1×10⁵ CAR-T cells and CD19⁺ Raji lymphoma cells were seeded at a ratio of 1:1 into a round-bottom 96-well plate, mixed thoroughly, centrifuged at room temperature (400g, 1 min) to promote cell-cell contact, and cultured in complete medium at 37°C for a certain period of time. Cell numbers were counted every 3-4 days. The results, shown in FIG. 1: k, indicate that B6I-28Z CAR-T cells demonstrated superior sustained proliferation compared with E28Z CAR-T and 28Z CAR-T cells.

1×10⁵ CAR-T cells and CD19⁺ Raji lymphoma cells were seeded at a ratio of 1:1 into a round-bottom 96-well plate, mixed thoroughly, and counted after 3 days. Half of the post-killing cells were transferred to a new round-bottom 96-well plate. Based on the counting results of the CAR-T cells, an equal number of CD19⁺ Raji lymphoma cells was added. After thorough mixing, the cells were counted again after 3 days. This process was repeated 6-7 times. As shown in FIG. 1: 1, B6I-28Z CAR-T cells displayed superior repetitive killing capacity compared with E28Z CAR-T and 28Z CAR-T cells.

At the late stage of repetitive killing assays, cells were collected, washed once with PBS, and stained with the following antibodies: anti-CD56-PE, anti-TIM3-PE, anti-PD1-APC, and anti-TIGIT-BV421. The result, shown in FIGs. 1: m-p, indicate B6I-28Z CAR-T cells exhibited reduced NK-like differentiation and decreased T cell exhaustion compared with E28Z CAR-T and 28Z CAR-T cells.

RNA was extracted from 28Z CAR-T, E28Z CAR-T, and B6I-28Z CAR-T cells after five rounds of repetitive killing and subjected to RNA-seq. GSEA analysis was performed on the sequencing results using a database related to NK-like differentiation. As shown in FIG. 1: q, B6I CAR-T cells showed the lowest degree of NK-like differentiation, whereas 28Z CAR-T cells exhibited the highest degree of NK-like differentiation.

RNA was extracted from 28Z CAR-T, E28Z CAR-T, and B6I-28Z CAR-T cells after five rounds of repetitive killing and subjected to RNA-seq. Through differential gene expression analysis results, as shown in FIG. 1: r, it was found that B6I CAR-T cells expressed higher levels of stemness-related transcription factors, such as KLF2.

A sterile suspension of Raji cells in PBS was prepared at a concentration of 3×10⁷ cells/ml. Each 6-week-old female B-NDG mouse was subcutaneously inoculated with 100 µL of 3×10⁶ Raji cells into the left back (Day 0). After 7 days, when the subcutaneous Raji tumors reached a diameter of 5-6 mm, mice were randomized into groups. Each mouse was injected via the tail vein with 100 µL of 6×10⁶ CAR-T cells. The model is shown in FIG. 1: s. Tumor size was measured regularly with a caliper, and tumor area was calculated as length × width. As shown in FIG. 1: t, tumor growth was fastest in the vector control group, followed by the 28Z CAR-T cell group. The B6I-28Z CAR-T cell group showed the strongest antitumor activity, while the E28Z CAR-T cell group exhibited a therapeutic effect between that of the 28Z and B6I-28Z CAR-T cell groups.

A sterile suspension of Raji cells in PBS was prepared at a concentration of 3×10⁷ cells/ml. Each 6-week-old female B-NDG mouse was subcutaneously inoculated with 100 µL of 3×10⁶ Raji cells into the left back (Day 0). After 7 days, when the subcutaneous Raji tumors reached a diameter of 5-6 mm, mice were randomized into groups. Each mouse was injected via the tail vein with 100 µL of 6×10⁶ CAR-T cells. Tumor size was measured regularly with a caliper, and tumor area was calculated as length × width. The results, shown on the left of FIG. 1: v, indicate that the tumors were completely eliminated after one month. Mice that achieved complete tumor clearance were selected. A sterile suspension of low-CD19-expressing K562 cells in PBS was prepared at a concentration of 1×10⁷ cells/ml. Each selected mouse was inoculated with 100 µL of the low-CD19-expressing K562 cell suspension into the right back to simulate tumor relapse. This relapse model is shown in FIG. 1: u. Tumor size was measured regularly with a caliper, and tumor area was calculated as length × width. The results, shown on the right of FIG. 1: v, indicate that the B6I-28Z CAR-T cell group exhibited the strongest therapeutic effect against the re-implanted low-antigen tumor, followed by the E28Z CAR-T cell group, while the 28Z CAR-T cell group exhibited the weakest therapeutic efficacy.

### Example 2

As shown in FIG. 2: a, the FMC63 scFv (anti-CD19) sequence in the anti-CD19 28Z CAR, E28Z CAR, and B6I-28Z CAR was respectively replaced with GC33 scFv (anti-GPC3), resulting in anti-GPC3 28Z CAR, anti-GPC3 E28Z CAR, and anti-GPC3 B6I-28Z CAR, respectively.

The nucleotide sequence of the Anti-GPC3 B6I-28Z CAR is as shown in SEQ ID NO: 9, specifically as follows:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCgatgttgtgatgactcagtctccactctccctgcccgtcacccctgga
gagccggcctccatctcctgcagatctagtcagagccttgtacacagtaatgccaacacctatttacattggtacctgcagaagc
cagggcagtctccacagctcctgatctataaagtttccaaccgattttctggggtccctgacaggttcagtggcagtggatcagg
cacagattttacactgaaaatcagcagagtggaggctgaggatgttggggtttattactgctctcaaaatacacatgttcctcctac
gtttggccaggggaccaagctggagatcaaacgtggtggaggcggttcaggcggaggtggctctggcggtggcggatcgca
ggtgcagctggtgcagtctggagctgaggtgaagaagcctggggcctcagtgaaggtctcctgcaaggcttctggatacacct
tcaccgactatgaaatgcactgggtgcgacaggcccctggacaagggcttgagtggatgggagctcttgatcctaaaactggt
gatactgcctacagtcagaagttcaagggcagagtcacgctgaccgcggacgaatccacgagcacagcctacatggagctga
gcagcctgagatctgaggacacggccgtgtattactgtacaagattctactcctatacttactggggccagggaaccctggtcac
cgtctcctcagcggccgcaattgaagttatgtatcctcctccttacctagacaatgagaagagcaatggaaccattatccatgtga
aagggaaacacctttgtccaagtcccctatttcccggaccttctaagcccttttgggtgctggtggtggttgggggagtcctggctt
gctatagcttgctagtaacagtggcctttattattttctgggtgaagaatagaaaAgccaaggcTaaAggcggagggGGT
GGCGGAgactatgagcccatccggaaaggccagcgggacctgtattctggcctgaatcagagacgcatcaggagtaag
aggagcaggctcctgcacagtgactacatgaacatgactccccgccgccccgggcccacccgcaagcattaccagccctatg
ccccaccacgcgacttcgcagcctatcgctccagagtgaagttcagcaggagcgcagacgcccccgcgtaccagcagggcc
agaaccagctctataacgagctcaatctaggacgaagagaggagtacgatgttttggacaagagacgtggccgggaccctga
gatggggggaaagccgagaaggaagaaccctcaggaaggcctgtacaatgaactgcagaaagataagatggcggaggcct
acagtgagattgggatgaaaggcgagcgccggaggggcaaggggcacgatggcctttaccagggtctcagtacagccacc
aaggacacctacgacgcccttcacatgcaggccctgccccctcgctaa.

Surface expression levels of CAR molecules on 28Z CAR-T, E28Z CAR-T, and B6I-28Z CAR-T cells were detected by flow cytometry using anti-HA rabbit primary antibody and anti-rabbit AF647 secondary antibody. The flow cytometry results shown in Figure 2: b indicate that B6I-28Z CAR-T cells restored the reduced surface CAR expression observed in E28Z CAR-T cells.

28Z CAR-T, E28Z CAR-T, and B6I-28Z CAR-T cells were detected by flow cytometry using anti-human CD4-PB and anti-human CD8-PE antibodies. The flow cytometry results, shown in FIG. 2: c, demonstrate that the ratios of CD4⁺ CAR-T cells to CD8⁺ CAR-T cells were similar among the three groups.

CAR-T cells were cultured without antigen stimulation for an extended period, and cell numbers were counted every 3-4 days. As shown in FIG. 2: d, B6I-28Z CAR-T and E28Z CAR-T cells exhibited better growth capacity than 28Z CAR-T cells.

### Comparative Example 1

As shown in FIGs. 3: a and 3: d, the FMC63 scFv (anti-CD19) sequence in the anti-CD19 28Z CAR, E28Z CAR, and B6I-28Z CAR was replaced with SS1 scFv (anti-mesothelin) and with anti-BCMA scFv, thereby generating CARs targeting mesothelin and CARs targeting BCMA, respectively. For the anti-mesothelin CARs, the CD3ε intracellular region contained linker sequences of 0, 3, 6, or 9 glycine residues, yielding the CARs: Anti-Meso B0I-28Z, Anti-Meso B3I-28Z, Anti-Meso B6I-28Z, and Anti-Meso B9I-28Z. For the anti-BCMA CARs, the CD3ε intracellular region contained linker sequences of 4, 5, 6, 7, or 8 glycine residues, yielding the CARs: Anti-BCMA B4I-28Z, Anti-BCMA B5I-28Z, Anti-BCMA B6I-28Z, Anti-BCMA B7I-28Z, and Anti-BCMA B8I-28Z.

The nucleotide sequence of Anti-Meso B0I-28Z is as shown in SEQ ID NO: 12, specifically:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCCAGGTACAACTGCAGCAGTCTGGGCCTGA
GCTGGAGAAGCCTGGCGCTTCAGTGAAGATATCCTGCAAGGCTTCTGGTTAC

TCATTCACTGGCTACACCATGAACTGGGTGAAGCAGAGCCATGGAAAGAGC
CTTGAGTGGATTGGACTTATTACTCCTTACAATGGTGCTTCTAGCTACAACCA
GAAGTTCAGGGGCAAGGCCACATTAACTGTAGACAAGTCATCCAGCACAGC
CTACATGGACCTCCTCAGTCTGACATCTGAAGACTCTGCAGTCTATTTCTGTG
CAAGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGCGG
TGGTGGATCGGACATCGAGCTCACTCAGTCTCCAGCAATCATGTCTGCATCT
CCAGGGGAGAAGGTCACCATGACCTGCAGTGCCAGCTCAAGTGTAAGTTAC
ATGCACTGGTACCAGCAGAAGTCAGGCACCTCCCCCAAAAGATGGATTTATG
ACACATCCAAACTGGCTTCTGGAGTCCCAGGTCGCTTCAGTGGCAGTGGGTC
TGGAAACTCTTACTCTCTCACAATCAGCAGCGTGGAGGCTGAAGATGATGCA
ACTTATTACTGCCAGCAGTGGAGTAAGCACCCTCTCACGTACGGTGCTGGGA
CAAAGTTGGAAATCAAAgcggccgcaattgaagttatgtatcctcctccttacctagacaatgagaagagcaat
ggaaccattatccatgtgaaagggaaacacctttgtccaagtcccctatttcccggaccttctaagcccttttgggtgctggtggtg
gttgggggagtcctggcttgctatagcttgctagtaacagtggcctttattattttctgggtgaagaatagaaaggccaaggccaa
ggactatgagcccatccggaaaggccagcgggacctgtattctggcctgaatcagagacgcatcaggagtaagaggagcag
gctcctgcacagtgactacatgaacatgactccccgccgccccgggcccacccgcaagcattaccagccctatgccccacca
cgcgacttcgcagcctatcgctccagagtgaagttcagcaggagcgcagacgcccccgcgtaccagcagggccagaacca
gctctataacgagctcaatctaggacgaagagaggagtacgatgttttggacaagagacgtggccgggaccctgagatgggg
ggaaagccgagaaggaagaaccctcaggaaggcctgtacaatgaactgcagaaagataagatggcggaggcctacagtga
gattgggatgaaaggcgagcgccggaggggcaaggggcacgatggcctttaccagggtctcagtacagccaccaaggaca
cctacgacgcccttcacatgcaggccctgccccctcgctaa.

The nucleotide sequence of Anti-Meso B3I-28Z is as shown in SEQ ID NO: 13, specifically:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC

CCTACGACGTGCCCGACTACGCCCAGGTACAACTGCAGCAGTCTGGGCCTGA
GCTGGAGAAGCCTGGCGCTTCAGTGAAGATATCCTGCAAGGCTTCTGGTTAC
TCATTCACTGGCTACACCATGAACTGGGTGAAGCAGAGCCATGGAAAGAGC
CTTGAGTGGATTGGACTTATTACTCCTTACAATGGTGCTTCTAGCTACAACCA
GAAGTTCAGGGGCAAGGCCACATTAACTGTAGACAAGTCATCCAGCACAGC
CTACATGGACCTCCTCAGTCTGACATCTGAAGACTCTGCAGTCTATTTCTGTG
CAAGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGCGG
TGGTGGATCGGACATCGAGCTCACTCAGTCTCCAGCAATCATGTCTGCATCT
CCAGGGGAGAAGGTCACCATGACCTGCAGTGCCAGCTCAAGTGTAAGTTAC
ATGCACTGGTACCAGCAGAAGTCAGGCACCTCCCCCAAAAGATGGATTTATG
ACACATCCAAACTGGCTTCTGGAGTCCCAGGTCGCTTCAGTGGCAGTGGGTC
TGGAAACTCTTACTCTCTCACAATCAGCAGCGTGGAGGCTGAAGATGATGCA
ACTTATTACTGCCAGCAGTGGAGTAAGCACCCTCTCACGTACGGTGCTGGGA
CAAAGTTGGAAATCAAAgcggccgcaattgaagttatgtatcctcctccttacctagacaatgagaagagcaat
ggaaccattatccatgtgaaagggaaacacctttgtccaagtcccctatttcccggaccttctaagcccttttgggtgctggtggtg
gttgggggagtcctggcttgctatagcttgctagtaacagtggcctttattattttctgggtgaagaatagaaaAgccaaggcTa
agggcggaggAgactatgagcccatccggaaaggccagcgggacctgtattctggcctgaatcagagacgcatcaggagt
aagaggagcaggctcctgcacagtgactacatgaacatgactccccgccgccccgggcccacccgcaagcattaccagccc
tatgccccaccacgcgacttcgcagcctatcgctccagagtgaagttcagcaggagcgcagacgcccccgcgtaccagcag
ggccagaaccagctctataacgagctcaatctaggacgaagagaggagtacgatgttttggacaagagacgtggccgggacc
ctgagatggggggaaagccgagaaggaagaaccctcaggaaggcctgtacaatgaactgcagaaagataagatggcggag
gcctacagtgagattgggatgaaaggcgagcgccggaggggcaaggggcacgatggcctttaccagggtctcagtacagcc
accaaggacacctacgacgcccttcacatgcaggccctgccccctcgctaa.

The amino acid sequence structure of Anti-BCMA B4I-28Z is: BCMA single-chain fragment variable (see patent CN109134665B) + CD28TM (SEQ ID NO: 5) + truncated CD3ε intracellular region (KNRKAKAKGGGGDYEPIRKGQRDLYSGLNQRRI, SEQ ID NO: 14) + CD28 intracellular region (SEQ ID NO: 2) + CD3ζ intracellular region (SEQ ID NO: 4).

The amino acid sequence structure of Anti-BCMA B5I-28Z is: BCMA single-chain fragment variable (see patent CN109134665B) + CD28TM (SEQ ID NO: 5) + truncated CD3ε intracellular region (KNRKAKAKGGGGGDYEPIRKGQRDLYSGLNQRRI, SEQ ID NO: 15) + CD28 intracellular region (SEQ ID NO: 2) + CD3ζ intracellular region (SEQ ID NO: 4).

The amino acid sequence structure of Anti-BCMA B6I-28Z is: BCMA single-chain fragment variable (see patent CN109134665B) + CD28TM (SEQ ID NO: 5) + truncated CD3ε intracellular region (SEQ ID NO: 1) + CD28 intracellular region (SEQ ID NO: 2) + CD3ζ intracellular region (SEQ ID NO: 4).

The nucleotide sequence of Anti-Meso B6I-28Z is as shown in SEQ ID NO: 19, specifically:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCCAGGTACAACTGCAGCAGTCTGGGCCTGA
GCTGGAGAAGCCTGGCGCTTCAGTGAAGATATCCTGCAAGGCTTCTGGTTAC
TCATTCACTGGCTACACCATGAACTGGGTGAAGCAGAGCCATGGAAAGAGC
CTTGAGTGGATTGGACTTATTACTCCTTACAATGGTGCTTCTAGCTACAACCA
GAAGTTCAGGGGCAAGGCCACATTAACTGTAGACAAGTCATCCAGCACAGC
CTACATGGACCTCCTCAGTCTGACATCTGAAGACTCTGCAGTCTATTTCTGTG
CAAGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGCGG
TGGTGGATCGGACATCGAGCTCACTCAGTCTCCAGCAATCATGTCTGCATCT
CCAGGGGAGAAGGTCACCATGACCTGCAGTGCCAGCTCAAGTGTAAGTTAC
ATGCACTGGTACCAGCAGAAGTCAGGCACCTCCCCCAAAAGATGGATTTATG
ACACATCCAAACTGGCTTCTGGAGTCCCAGGTCGCTTCAGTGGCAGTGGGTC
TGGAAACTCTTACTCTCTCACAATCAGCAGCGTGGAGGCTGAAGATGATGCA
ACTTATTACTGCCAGCAGTGGAGTAAGCACCCTCTCACGTACGGTGCTGGGA
CAAAGTTGGAAATCAAAgcggccgcaattgaagttatgtatcctcctccttacctagacaatgagaagagcaat
ggaaccattatccatgtgaaagggaaacacctttgtccaagtcccctatttcccggaccttctaagcccttttgggtgctggtggtg
gttgggggagtcctggcttgctatagcttgctagtaacagtggcctttattattttctgggtgaagaatagaaaAgccaaggcTa
aAggcggagggGGTGGCGGAgactatgagcccatccggaaaggccagcgggacctgtattctggcctgaatcaga
gacgcatcaggagtaagaggagcaggctcctgcacagtgactacatgaacatgactccccgccgccccgggcccacccgca
agcattaccagccctatgccccaccacgcgacttcgcagcctatcgctccagagtgaagttcagcaggagcgcagacgcccc
cgcgtaccagcagggccagaaccagctctataacgagctcaatctaggacgaagagaggagtacgatgttttggacaagaga
cgtggccgggaccctgagatggggggaaagccgagaaggaagaaccctcaggaaggcctgtacaatgaactgcagaaag
ataagatggcggaggcctacagtgagattgggatgaaaggcgagcgccggaggggcaaggggcacgatggcctttaccag
ggtctcagtacagccaccaaggacacctacgacgcccttcacatgcaggccctgccccctcgctaa.

The amino acid sequence structure of Anti-BCMA B7I-28Z is: BCMA single-chain fragment variable (see patent CN109134665B) + CD28TM (SEQ ID NO: 5) + truncated CD3ε intracellular region (KNRKAKAKGGGGGGGDYEPIRKGQRDLYSGLNQRRI, SEQ ID NO: 16) + CD28 intracellular region (SEQ ID NO: 2) + CD3ζ intracellular region (SEQ ID NO: 4).

The amino acid sequence structure of Anti-BCMA B8I-28Z is: BCMA single-chain fragment variable (see patent CN109134665B) + CD28TM (SEQ ID NO: 5) + truncated CD3ε intracellular region
(KNRKAKAKGGGGGGGGDYEPIRKGQRDLYSGLNQRRI, SEQ ID NO: 17) + CD28 intracellular region (SEQ ID NO: 2) + CD3ζ intracellular region (SEQ ID NO: 4).

The nucleotide sequence of Anti-Meso B9I-28Z is as shown in SEQ ID NO: 18, specifically:

ATGGTGAGCAAGGGCGAGGAGCTGTTCACCGGGGTGGTGCCCATCCTGGT
CGAGCTGGACGGCGACGTAAACGGCCACAAGTTCAGCGTGTCCGGCGAGGG
CGAGGGCGATGCCACCTACGGCAAGCTGACCCTGAAGTTCATCTGCACCACC
GGCAAGCTGCCCGTGCCCTGGCCCACCCTCGTGACCACCCTGACCTACGGCG
TGCAGTGCTTCAGCCGCTACCCCGACCACATGAAGCAGCACGACTTCTTCAA
GTCCGCCATGCCCGAAGGCTACGTCCAGGAGCGCACCATCTTCTTCAAGGAC
GACGGCAACTACAAGACCCGCGCCGAGGTGAAGTTCGAGGGCGACACCCTG
GTGAACCGCATCGAGCTGAAGGGCATCGACTTCAAGGAGGACGGCAACATC
CTGGGGCACAAGCTGGAGTACAACTACAACAGCCACAACGTCTATATCATGG
CCGACAAGCAGAAGAACGGCATCAAGGTGAACTTCAAGATCCGCCACAACA
TCGAGGACGGCAGCGTGCAGCTCGCCGACCACTACCAGCAGAACACCCCCA
TCGGCGACGGCCCCGTGCTGCTGCCCGACAACCACTACCTGAGCACCCAGTC
CGCCCTGAGCAAAGACCCCAACGAGAAGCGCGATCACATGGTCCTGCTGGA
GTTCGTGACCGCCGCCGGGATCACTCTCGGCATGGACGAGCTGTACAAGGGA
AGCGGAGAGGGCAGAGGAAGTCTGCTAACATGCGGTGACGTCGAGGAGAAT
CCTGGACCTatgcttctcctggtgacaagccttctgctctgtgagttaccacacccagcattcctcctgatcccaTACC
CCTACGACGTGCCCGACTACGCCCAGGTACAACTGCAGCAGTCTGGGCCTGA
GCTGGAGAAGCCTGGCGCTTCAGTGAAGATATCCTGCAAGGCTTCTGGTTAC
TCATTCACTGGCTACACCATGAACTGGGTGAAGCAGAGCCATGGAAAGAGC
CTTGAGTGGATTGGACTTATTACTCCTTACAATGGTGCTTCTAGCTACAACCA
GAAGTTCAGGGGCAAGGCCACATTAACTGTAGACAAGTCATCCAGCACAGC
CTACATGGACCTCCTCAGTCTGACATCTGAAGACTCTGCAGTCTATTTCTGTG
CAAGGGGGGGTTACGACGGGAGGGGTTTTGACTACTGGGGCCAAGGGACCA
CGGTCACCGTCTCCTCAGGTGGAGGCGGTTCAGGCGGCGGTGGCTCTAGCGG
TGGTGGATCGGACATCGAGCTCACTCAGTCTCCAGCAATCATGTCTGCATCT
CCAGGGGAGAAGGTCACCATGACCTGCAGTGCCAGCTCAAGTGTAAGTTAC
ATGCACTGGTACCAGCAGAAGTCAGGCACCTCCCCCAAAAGATGGATTTATG
ACACATCCAAACTGGCTTCTGGAGTCCCAGGTCGCTTCAGTGGCAGTGGGTC

TGGAAACTCTTACTCTCTCACAATCAGCAGCGTGGAGGCTGAAGATGATGCA
ACTTATTACTGCCAGCAGTGGAGTAAGCACCCTCTCACGTACGGTGCTGGGA
CAAAGTTGGAAATCAAAgcggccgcaattgaagttatgtatcctcctccttacctagacaatgagaagagcaat
ggaaccattatccatgtgaaagggaaacacctttgtccaagtcccctatttcccggaccttctaagcccttttgggtgctggtggtg
gttgggggagtcctggcttgctatagcttgctagtaacagtggcctttattattttctgggtgaagaatagaaaAgccaaggcTa
aAggcggaggTGGTGGCGGAggAggtggagactatgagcccatccggaaaggccagcgggacctgtattctgg
cctgaatcagagacgcatcaggagtaagaggagcaggctcctgcacagtgactacatgaacatgactccccgccgccccgg
gcccacccgcaagcattaccagccctatgccccaccacgcgacttcgcagcctatcgctccagagtgaagttcagcaggagc
gcagacgcccccgcgtaccagcagggccagaaccagctctataacgagctcaatctaggacgaagagaggagtacgatgttt
tggacaagagacgtggccgggaccctgagatggggggaaagccgagaaggaagaaccctcaggaaggcctgtacaatga
actgcagaaagataagatggcggaggcctacagtgagattgggatgaaaggcgagcgccggaggggcaaggggcacgat
ggcctttaccagggtctcagtacagccaccaaggacacctacgacgcccttcacatgcaggccctgccccctcgctaa.

As shown in FIG. 3, the structures of the anti-mesothelin 28Z CAR, E28Z CAR, B0I-28Z CAR, B3I-28Z CAR, B6I-28Z CAR, and B9I-28Z CAR are illustrated in FIG. 3: a.

Surface expression levels of CAR molecules on 28Z CAR-T, E28Z CAR-T, B0I-28Z CAR-T, B3I-28Z CAR-T, B6I-28Z CAR-T, and B9I-28Z CAR-T cells were detected by flow cytometry using anti-HA rabbit primary antibody and anti-rabbit AF647 secondary antibody. The flow cytometry results are shown in FIG. 3: b, and the statistical results are shown in FIG. 3: c. The results indicate that B0I-28Z, B3I-28Z, B6I-28Z, and B9I-28Z CAR-T cells can partially restore the reduced surface CAR expression observed in E28Z CAR-T cells, with B6I-28Z CAR-T cells demonstrating the most effective restoration.

The structures of the anti-BCMA 28Z CAR, E28Z CAR, B4I-28Z CAR, B5I-28Z CAR, B6I-28Z CAR, B7I-28Z CAR, and B8I-28Z CAR are shown in FIG. 3: d.

Surface expression levels of CAR modules on B4I-28Z CAR-T, B5I-28Z CAR-T, B6I-28Z CAR-T, B7I-28Z CAR-T, and B8I-28Z CAR-T cells were detected by flow cytometry using anti-HA rabbit primary antibody and anti-rabbit AF647 secondary antibody. The flow cytometry results, shown in FIG. 3: e, indicate that B6I-28Z CAR-T cells exhibited the highest surface CAR expression levels.

Using the mesothelin-positive solid tumor cell line HCT116 as target cells, the aforementioned *in vitro* repetitive killing assay was performed with the anti-mesothelin B6I-28Z CAR-T cells. The CAR-T cell numbers were monitored over time. As shown in FIG. 3: f, Anti-Mesothelin B6I-28Z CAR-T cells exhibited superior sustained proliferative capacity compared with conventional 28Z CAR-T cells.

A subcutaneous tumor model was established by injecting HCT116 tumor cells into mice. Five days later, CAR-T cells were injected. As shown in FIG. 3: g, the results demonstrate that Anti-Mesothelin B6I-28Z CAR-T cells achieved better tumor control effect than 28Z CAR-T cells and effectively prolonged the survival time of the mice.

### Comparative Example 2

As shown in FIG. 4: a, based on the anti-CD19 B6I-28Z CAR structure described above, the CD28 costimulatory signaling region was replaced with the 4-1BB costimulatory signaling region to generate a BBZ-based B6I CAR, designated as Anti-CD19 B6I-BBZ CAR.

The amino acid sequence structure of Anti-CD19 B6I-BBZ CAR is: CD19 single-chain fragment variable (SEQ ID NO: 10) + CD8TM (SEQ ID NO: 5) + truncated CD3ε intracellular region (KNRKAKAKGGGGGGGDYEPIRKGQRDLYSGLNQRRI, SEQ ID NO: 16) + 4-1BB intracellular region (SEQ ID NO: 4) + CD3ζ intracellular region (SEQ ID NO: 4).

The amino acid sequence of Anti-CD19 B6I-BBZ CAR is as shown in SEQ ID NO: 22, specifically:

MALPVTALLLPLALLLHAARPYPYDVPDYADIQMTQTTSSLSASLGDRVTISC
RASQDISKYLNWYQQKPDGTVKLLIYHTSRLHSGVPSRFSGSGSGTDYSLTISNL
EQEDIATYFCQQGNTLPYTFGGGTKLEITGGGGSGGGGSGGGGSEVKLQESGPG
LVAPSQSLSVTCTVSGVSLPDYGVSWIRQPPRKGLEWLGVIWGSETTYYNSALK
SRLTIIKDNSKSQVFLKMNSLQTDDTAIYYCAKHYYYGGSYAMDYWGQGTSVT
VSSASTTTPAPRPPTPAPTIASQPLSLRPEACRPAAGGAVHTRGLDFACDIYIWAP
LAGTCGVLLLSLVITLYCKNRKAKAKGGGGGGDYEPIRKGQRDLYSGLNQRRIK
RGRKKLLYIFKQPFMRPVQTTQEEDGCSCRFPEEEEGGCELRVKFSRSADAPAY
QQGQNQLYNELNLGRREEYDVLDKRRGRDPEMGGKPRRKNPQEGLYNELQKD
KMAEAYSEIGMKGERRRGKGHDGLYQGLSTATKDTYDALHMQALPPR*.

The nucleotide sequence of Anti-CD19 B6I-BBZ CAR is as shown in SEQ ID NO: 23, specifically:

ATGGCCTTACCAGTGACCGCCTTGCTCCTGCCGCTGGCCTTGCTGCTCCAC
GCCGCCAGGCCGTACCCCTACGACGTGCCCGACTACGCCGACATCCAGATGA
CACAGACTACATCCTCCCTGTCTGCCTCTCTGGGAGACAGAGTCACCATCAG
TTGCAGGGCAAGTCAGGACATTAGTAAATATTTAAATTGGTATCAGCAGAAA
CCAGATGGAACTGTTAAACTCCTGATCTACCATACATCAAGATTACACTCAG
GAGTCCCATCAAGGTTCAGTGGCAGTGGGTCTGGAACAGATTATTCTCTCAC
CATTAGCAACCTGGAGCAAGAAGATATTGCCACTTACTTTTGCCAACAGGGT
AATACGCTTCCGTACACGTTCGGAGGGGGGACCAAGCTGGAGATCACAGGT
GGCGGTGGCTCGGGCGGTGGTGGGTCGGGTGGCGGCGGATCTGAGGTGAAA
CTGCAGGAGTCAGGACCTGGCCTGGTGGCGCCCTCACAGAGCCTGTCCGTCA
CATGCACTGTCTCAGGGGTCTCATTACCCGACTATGGTGTAAGCTGGATTCG
CCAGCCTCCACGAAAGGGTCTGGAGTGGCTGGGAGTAATATGGGGTAGTGA
AACCACATACTATAATTCAGCTCTCAAATCCAGACTGACCATCATCAAGGAC
AACTCCAAGAGCCAAGTTTTCTTAAAAATGAACAGTCTGCAAACTGATGACA
CAGCCATTTACTACTGTGCCAAACATTATTACTACGGTGGTAGCTATGCTATG
GACTACTGGGGCCAAGGAACCTCAGTCACCGTCTCCTCAGCCAGTACCACCA
CTCCCGCACCCCGCCCTCCTACTCCTGCCCCTACCATTgccagtCAACCGCTTAG

TCTGAGACCTGAGGCCTGTAGGCCCGCTGCTGGTGGCGCTGTGCACACCCGA
GGATTGGACTTCGCTTGCGACATCTACATCTGGGCACCTCTGGCTGGGACCT
GCGGCGTGTTGTTGTTGAGCCTGGTGATTACGCTGTACTGTaagaatagaaaAgccaa
ggcTaaAggcggagggGGTGGCGGAgactatgagcccatccggaaaggccagcgggacctgtattctggcctga
atcagagacgcatcaaacggggcagaaagaaactcctgtatatattcaaacaaccatttatgagaccagtacaaactactcaag
aggaagatggctgtagctgccgatttccagaagaagaagaaggaggatgtgaactgagagtgaagttcagcaggagcgcag
acgcccccgcgtaccagcagggccagaaccagctctataacgagctcaatctaggacgaagagaggagtacgatgttttgga
caagagacgtggccgggaccctgagatggggggaaagccgagaaggaagaaccctcaggaaggcctgtacaatgaactg
cagaaagataagatggcggaggcctacagtgagattgggatgaaaggcgagcgccggaggggcaaggggcacgatggcc
tttaccagggtctcagtacagccaccaaggacacctacgacgcccttcacatgcaggccctgccccctcgctaa.

When performing stimulation with low-CD19-expressing K562 cells, Anti-CD19 B6I-BBZ CAR-T cells exhibited stronger downstream signaling, including increased pERK and pS6, indicating enhanced antigen sensitivity, as shown in FIG. 4: b. The aforementioned CAR-T cells were used to kill K562 cells expressing low or high levels of CD19. Tumor cell numbers were counted at 24 h, 48 h, and 72 h, respectively. As shown in FIG. 4: c, Anti-CD19 B6I-BBZ CAR-T cells more effectively eliminated the tumor cells.

Using naturally CD19-positive Raji cells as target cells, the *in vitro* repetitive killing assay was performed, and the proliferation of CAR-T cells during repetitive killing cycles was monitored. As shown in FIG. 4: d, CD19 B6I-BBZ CAR-T cells demonstrated markedly improved sustained proliferative capacity. After eight rounds of repetitive killing, CAR-T cells were collected, and the NK-like differentiation marker CD56 was detected. As shown in FIG. 4: e, B6I-BBZ CAR-T cells exhibited weaker NK-like differentiation, indicating better functional persistence.

To simulate *in vivo* tumor antigen heterogeneity, equal numbers of low-CD19-expressing K562 tumor cells and high-CD19-expressing K562 tumor cells were mixed and subcutaneously injected into mice to establish a xenograft tumor model. Five days later, CAR-T cells were injected. The B6I-BBZ CAR-T cell group exhibited a superior tumor control effect, as shown in FIG. 4: f, and effectively prolonged the survival time of the mice, as shown in FIG. 4: g.

The above are only some preferred embodiments of the present disclosure instead of limitations on the present disclosure in any form or substance. It should be noted that, for those skilled in the art, various modifications and additions may be made without departing from the method of the present disclosure, and all such modifications and additions shall also fall within the protection scope of the present disclosure. The equivalent changes of alternations, modifications and evolutions can be made by those skilled in the art using the technical contents revealed above and without departing from the spirit and scope of the present disclosure, and those equivalent changes are regarded as equivalent embodiments of the present disclosure. Meanwhile, any alterations, modifications, and evolutions of any equivalent changes made to the above embodiments according to the essential technical concepts of the present disclosure still fall within the scope of the technical solution of the present disclosure.

## Claims

1. A chimeric antigen receptor, comprising:
an extracellular domain, a transmembrane domain, and an intracellular domain, which are sequentially connected;
wherein the extracellular domain comprises an antigen-binding region and a hinge region; and
wherein the intracellular domain comprises, in sequence, a truncated CD3ε intracellular region, a costimulatory signaling region, and a CD3ζ intracellular region.

2. The chimeric antigen receptor according to claim 1, wherein, compared to a CD3ε intracellular region, the truncated CD3ε intracellular region consists of a BRS sequence, a linker sequence, and an ITAM sequence linked in sequence.

3. The chimeric antigen receptor according to claim 2, wherein the linker sequence comprises a plurality of amino acid residues; the amino acid residues of the linker sequence comprise glycine and/or serine; further, the number of the amino acid residues in the linker sequence is in a range of 0-9; and more further, an amino acid sequence of the truncated CD3ε intracellular region is as shown in SEQ ID NO: 1.

4. The chimeric antigen receptor according to claim 1, wherein the costimulatory signaling region is one or more of intracellular regions of CD27, CD28, CD134, 4-1BB, and ICOS.

5. The chimeric antigen receptor according to claim 4, wherein the chimeric antigen receptor further comprises one or more of the following:
(1) an amino acid sequence of CD28 intracellular region is as shown in SEQ ID NO: 2;
(2) an amino acid sequence of 4-1BB intracellular region is as shown in SEQ ID NO: 3;
(3) an amino acid sequence of CD3ζ intracellular region is as shown in SEQ ID NO: 4.

6. The chimeric antigen receptor according to claim 1, wherein the chimeric antigen receptor further comprises one or more of the following:
a. the antigen-binding region is a single-chain fragment variable targeting tumor surface antigen, wherein the single-chain fragment variable is one or more of CD19-, GPC3-, mesothelin-, CD20-, CD22-, CD123-, CD30-, CD33-, CD38-, CD138-, BCMA-, fibroblast activation protein-, CEA-, EGFRvIII-, PSMA-, Her2-, IL13Rα2-, CD171-, and GD2-binding scFvs;
b. the transmembrane domain is CD28TM, CD4, CD8α, OX40, or H2-Kb.

7. The chimeric antigen receptor according to claim 6, wherein the chimeric antigen receptor further comprises one or more of the following:
c. an amino acid sequence of the single-chain fragment variable is as shown in SEQ ID NO: 10, SEQ ID NO: 11, or SEQ ID NO: 20;
d. the transmembrane domain is CD28TM, and an amino acid sequence of CD28TM is as shown in SEQ ID NO: 5.

8. The chimeric antigen receptor according to any one of claims 1-7, wherein an amino acid sequence of the chimeric antigen receptor is as shown in SEQ ID NO: 6, SEQ ID NO: 7, SEQ ID NO: 21, or SEQ ID NO: 22.

9. A polynucleotide sequence, comprising:
(1) a polynucleotide sequence encoding the chimeric antigen receptor according to any one of claims 1-8; and/or
(2) a complementary sequence of the polynucleotide sequence described in (1).

10. The polynucleotide sequence according to claim 9, wherein the polynucleotide sequence is as shown in SEQ ID NO: 8, SEQ ID NO: 9, SEQ ID NO: 19, or SEQ ID NO: 23.

11. A nucleic acid construct, comprising the polynucleotide sequence according to any one of claims 9-10;
preferably, the nucleic acid construct is a vector;
more preferably, the nucleic acid construct is a lentiviral vector comprising an origin of replication, a 3' LTR, a 5' LTR, and the polynucleotide sequence according to any one of claims 9-10.

12. A lentiviral vector system, comprising the nucleic acid construct according to claim 11 and a lentiviral auxiliary component.

13. A genetically modified T cell, comprising the polynucleotide sequence according to any one of claims 9-10 or the nucleic acid construct according to claim 11, or being infected with the lentiviral vector system according to claim 12.

14. The chimeric antigen receptor according to any one of claims 1-8, or the polynucleotide sequence according to any one of claims 9-10, or the nucleic acid construct according to claim 11, or the lentiviral vector system according to claim 12, for use in the preparation of a product for one or more of the following purposes: (1) preparing CAR-T cells; (2) increasing surface expression level of CAR on CAR-T cells; (3) enhancing antigen sensitivity of CAR-T cells; (4) suppressing IFN-γ, IL-2, and TNF cytokine secretion of CAR-T cells; (5) promoting natural growth of CAR-T cells under resting conditions; (6) enhancing sustained proliferation of CAR-T cells after stimulation by target cells; (7) improving repetitive killing capacity of CAR-T cells; (8) reducing NK-like differentiation and exhaustion of CAR-T cells; (9) increasing stemness and memory cell subsets of CAR-T cells.

15. The chimeric antigen receptor according to any one of claims 1-8, or the polynucleotide sequence according to any one of claims 9-10, or the nucleic acid construct according to claim 11, or the lentiviral vector system according to claim 12, or the genetically modified T cell according to claim 13, for use in the preparation of a product for tumor therapy.

16. A method for treating a tumor, comprising administering to a subject in need thereof a therapeutically effective amount of the genetically modified T cell according to claim 13.

17. The method according to claim 16, wherein the tumor is B-cell lymphoma, mantle cell lymphoma, acute lymphoblastic leukemia, chronic lymphocytic leukemia, hairy cell leukemia, acute myeloid leukemia, hepatocellular carcinoma, melanoma, ovarian clear cell carcinoma, or lung squamous cell carcinoma.
